(19) Europäisches Patentamt / European Patent Office / Office européen des brevets

(11) **EP 3 582 715 B1**

(12) **EUROPEAN PATENT SPECIFICATION**

(45) Date of publication and mention
of the grant of the patent:
**27.10.2021 Bulletin 2021/43**

(21) Application number: **18709399.2**

(22) Date of filing: **15.02.2018**

(51) Int Cl.:
***A61C 8/00*** *(2006.01)*

(86) International application number:
**PCT/IB2018/050932**

(87) International publication number:
**WO 2018/150350 (23.08.2018 Gazette 2018/34)**

(54) **A METHOD AND SYSTEM OF IDENTIFYING A DENTAL IMPLANT FOR AN OPTIMIZED IMPLANT SITE**

VERFAHREN UND SYSTEM ZUR IDENTIFIZIERUNG EINES ZAHNIMPLANTATS FÜR EINE OPTIMIERTE IMPLANTATIONSSTELLE

PROCÉDÉ ET SYSTÈME D'IDENTIFICATION D'UN IMPLANT DENTAIRE POUR UN SITE D'IMPLANT OPTIMISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2017 IT 201700017978**

(43) Date of publication of application:
**25.12.2019 Bulletin 2019/52**

(73) Proprietor: **Emanuelli, Silvio Franco**
**18038 Sanremo (IT)**

(72) Inventors:
• **MANES, Federico**
  **18038 Sanremo (IMPERIA) (IT)**
• **EMANUELLI, Silvio Franco**
  **18038 Sanremo (IMPERIA) (IT)**

(74) Representative: **Penza, Giancarlo**
**Bugnion S.p.A.**
**Viale Lancetti, 17**
**20158 Milano (IT)**

(56) References cited:
US-A1- 2009 111 071    US-A1- 2011 136 080
US-A1- 2012 203 366    US-A1- 2012 239 364

## Description

### FIELD OF THE INVENTION

The present invention relates to a computer-implemented method of identifying a dental implant for an implant site.

The present invention further relates to a system of identifying a dental implant for an implant site.

In particular, the present invention relates to a method and/or system of identifying an optimized dental implant couplable to an implant site.

More particularly, the present invention relates to a method and/or system of identifying an optimiseddental implant to be applied to a maxillary arch of a patient's oral cavity as a result of a missing tooth, wherein the following description refers to this field of application for the sole purpose of simplifying the inventive exposure.

It is understood that an identification of an optimised dental implant in connection with another area of the oral cavity and with any type of missing tooth, falls within the scope of the present invention.

### BACKGROUND OF THE INVENTION

At present, dental implants are designed and prepared based on the dentist's instructions basically deriving from the experience gained. Some guidelines recommend rather trivial criteria for defining the characteristic parameters of dental implants to be applied to patients.

In particular, the assessment on the suitability to receive the implant on the part of the patient, notably as to whether the patient's maxillary bone is capable or uncapable of supporting the dental implant as such, as well as the evaluation on the positioning of said dental implant, are left to subjective and empirical considerations rather than to a reliable technical-structural analysis of both the favourite implant and the anatomical structures involved.

US 2012/239364 A1 discloses a computer-implemented method of identifying, in a patient, at least one dental implant for an implant site, and an identification system of at least one dental implant for an implant site. However, it does not disclose that one or more dental implants are identified from a database to suit an optimised dental implant site.

It is an object of the present invention to provide a method of identifiying an optimised dental implant intended for an implant site.

Another object of the present invention is to provide a method of identifiying an optimised dental implant adapted to ensure a durable and effective dental implant. in particular upon changing of implementation specific parameters.

Another object of the present invention is to provide an easy-to-implement method of identifiying an optimised dental implant.

## SUMMARY OF THE INVENTION

In a first aspect, the present invention discloses a computer-implemented method of identifiying a dental implant intended for an implant site as described in claim 1.

Further advantageous aspects of the method are disclosed in the dependent claims 2 to 10.

In a third aspect, the present invention discloses a system of identifiying a dental implant intended for an implant site as described in claim 12.

In a fourth aspect, the present invention discloses a dental implant obtained by the method of the first aspect or the system of the third aspect of the invention as described in claim 21.

Further beneficial aspects of the system are described in the dependent claims 13 to 20.

In a fifth aspect, the present invention discloses a computer program configured to implement the method of the second aspect, as described in claim 11.

The invention confers the main technical effect arising from identifying an optimised dental implant in terms of sizing and location.

The invention as described herein, particularly achieves the technical effects of:

- identifying an exact dental implant in terms of structure and sizing.
- identifying an objective dental implant in terms of design.
- identifying a durable and effective dental implant.
- identifying the dental implant in an easy manner.
- identifying an optimised dental implant in accordance with guidelines, and suitable for the anatomy and proposed treatment with respect to available optimised dental implants.

It should be noted that the simulation system/method of the invention can not be performed by purely mental or mathematical means, nor by the thought process that led to the simulation method.

The simulation performed by the invention achieves technical functions typical of modern engineering work. It provides for realistic prediction of the performance of a dental implant in respect to the designed implant site which shall accommodate the former, and thereby ideally allows the dental implant to be developed so accurately such that a prototype's chances of success can be assessed before it is built.

The technical result increases with the speed of the simulation method, as this enables a wide range of designs to be virtually tested and examined for suitability before the expensive implant fabrication process starts. Without a suitable technical support, an advanced test on a complex dental implant and/or a careful selection among many different designs would not be possible, or at least not feasible within a reasonable time.

It follows that computer-implemented simulation methods for virtual testing represent a practical and practice-oriented part of the dentist's or healthcare professional's or treatment provider's toolkit. What makes them so important is the fact that there isn't a purely mathematical, theoretical or mental method which is capable of providing a complete and/or fast prediction of a dental implant performance according to the parameters of the invention that is different or diversifiable for each single patient. The technical effects and/or the advantages already mentioned, as well as further technical effects and/or advantages of the invention, will become more apparent from the description made hereafter of an illustrative embodiment described by way of non-limiting example with reference to the accompanying drawings.

## BRIEF DESCRIPTION OF THE DRAWINGS

Figure 1 is a schematic view of a dental implant in a maxillary arch according to the present invention.
Figure 2 shows a screenshot of a software simulation of a dental prosthesis and maxillary arch thereof in top plan view;
Figure 3 shows a screenshot of a software simulation of a dental prosthesis and maxillary arch thereof in sectional view along a plane transverse to a foreseen dental prosthesis.
Figure 4 is a schematic view of the sectional view of Figure 2.
Figure 5 is a schematic view of the sectional view of Figure 3.
Figure 6 is a more detailed schematic view of Figure 5.

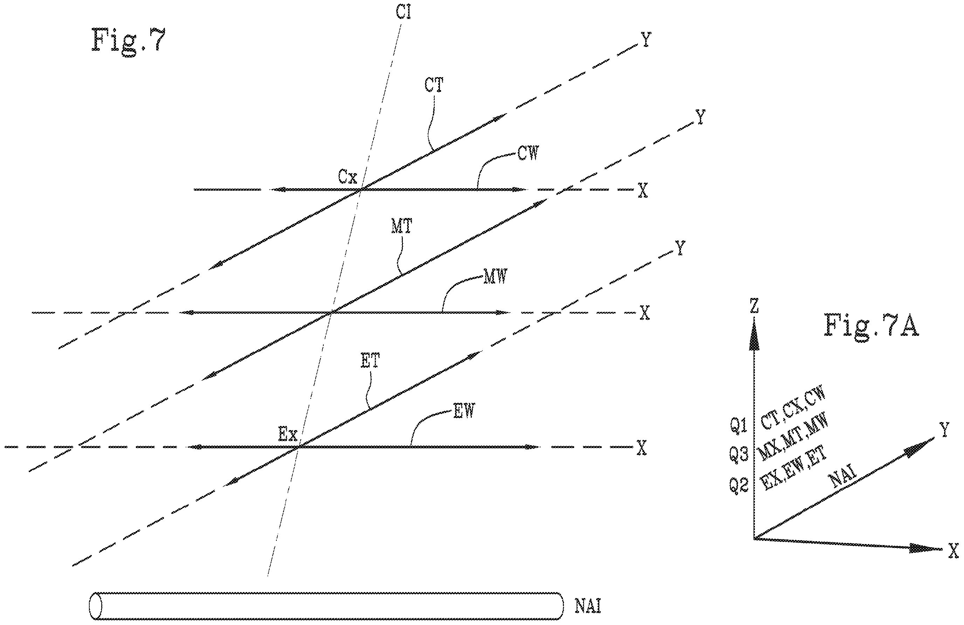

Figure 7 is a schematic view of representative mandibular bone measures with respect to an ideal surgical axis.
Figure 7A shows a reference system for the measures of Figure 7.
Figure 8 is a view of a representative volume of an implant site for the dental implant of Figure 1.
Figure 9 is a schematic representation of reciprocal positions of ideal surgical axes and a prosthetic axis.
Figure 10A, 10B and 10C are schematic views for the evaluation of representative parameters of an optimised dental implant according to the invention.
Figure 11 is a block diagram of some steps of the inventive method.
Figure 12 is a schematic plan view of incisor teeth.

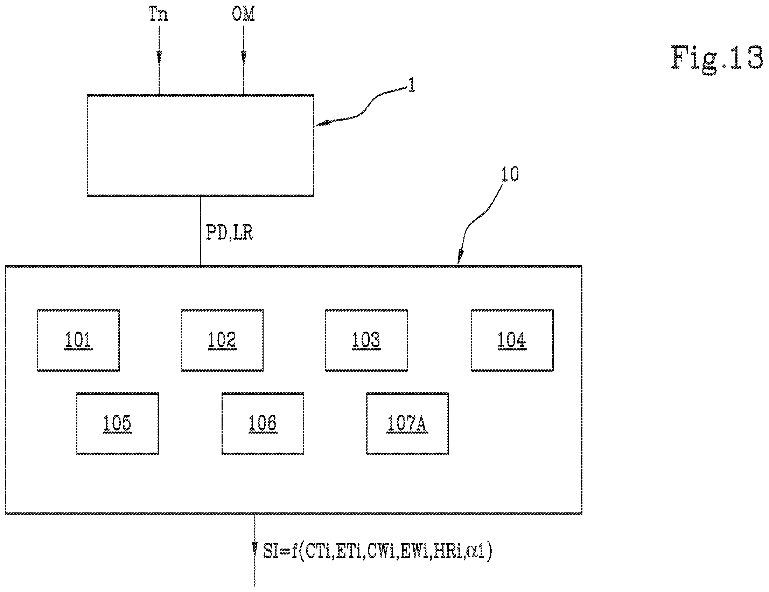

Figure 13 is a block diagram of the optimised implant site simulation system.
Figure 14 is a schematic block diagram of a dental implant identification system.
Figure 15 is a schematic view of a hypothetical positioning of dental implants as a function of the position of the dental elements to be replaced.

## DETAILED DESCRIPTION

The invention describes a method and system of identifying a dental implant intended for an optimised implant site which actuates a simulation of a patient's damaged oral cavity and defines the most suited system to be applied, starting from an implant site capable of receiving a dental implant aimed at repairing the damage observed.

The invention further allows identifying a dental problem as well as the optimised dental implant adapted to overcome the problems highlighted. The terminology as described hereinafter will be used several times and maintained as a reference in the appended claims as follows:

dental implant = screw iserted into the mandibular and/or maxillary bone;
implant site = cavity afforded within the mandible and/or maxilla which accomodates the dental implant;
abutment = pin which is coupled to the free end of the dental implant; crown = tooth-shaped cover coupled to the free end of the abutment; dental prosthesis = set of abutment and crown;
edentulous site = volumetric space intended to receive the dental prosthesis, in particular space between two existing teeth;
vestibule = space between the cheeks and the gingiva;
mandible = bone which forms the lower scaffold of the mouth; it accomodates the lower teeth in the maxillary arch and is the only movable part of the face.
maxilla = bone which forms the upper scaffold of the mouth and accomodates the upper dental arch.

Unlike the mandible, the maxilla is a fixed bone, which does not move with the opening and closing of the mouth.

Overall, the two bones are also referred to as maxillary bones and in the present description the term maxillary bone (OM), will be used in this respect, i.e. without excluding that the example according to which a dental prosthesis is applied to the lower arch, excludes application to the upper arch.

In the description, it will be illustrated by way of example the case of a dental prosthesis PD applied in replacement of a premolar tooth in the maxillary arch, without the invention having to be construed as limited to this example; indeed the method herein described may be also applied where other teeth, such as molars, canines, incisors etc. are to be replaced and moreover on a different arch.

In a first aspect, the invention describes a simulation method of an implant site.

The simulation performed by the invention achieves technical functions typical of modern engineering work. It provides for realistic prediction of the performance of a dental implant in respect to the designed implant site which shall accommodate the former, and thereby ideally allows the dental implant to be developed so accurately such that a prototype's chances of success can be assessed before it is built.

The technical result increases with the speed of the simulation method, as this enables a wide range of designs to be virtually tested and examined for suitability before the expensive implant fabrication process starts. Without a suitable technical support, an advanced test on a complex dental implant and/or a careful selection among many different designs would not be possible, or at least not feasible within a reasonable time.

It follows that computer-implemented simulation methods for virtual testing represent a practical and practice-oriented part of the dentist's or healthcare professional's or treatment provider's toolkit. What makes them so important is the fact that that there isn't a purely mathematical, theoretical or mental method which is capable of providing a complete and / or fast prediction of a dental implant performance according to the parameters of the invention that is different or diversifiable for each single patient.

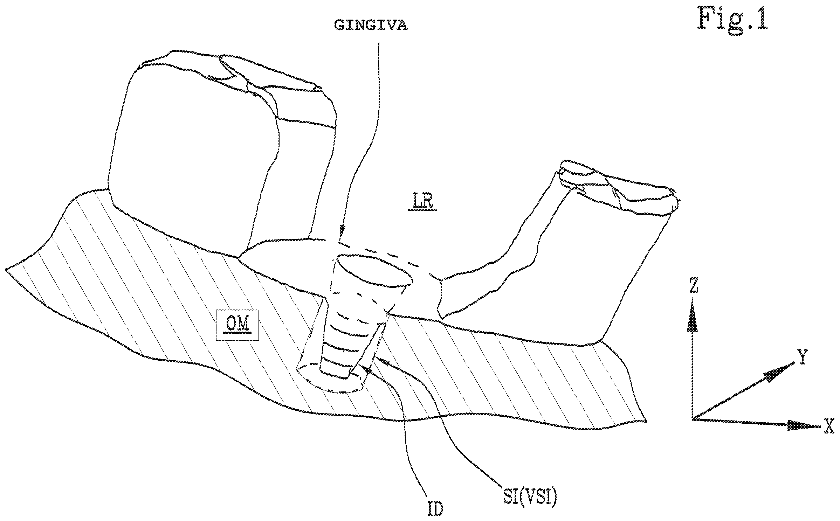

With particular reference to Figures 1 to 5, the simulation method of an implant site Slprovides an initial step f1 of graphic simulation.

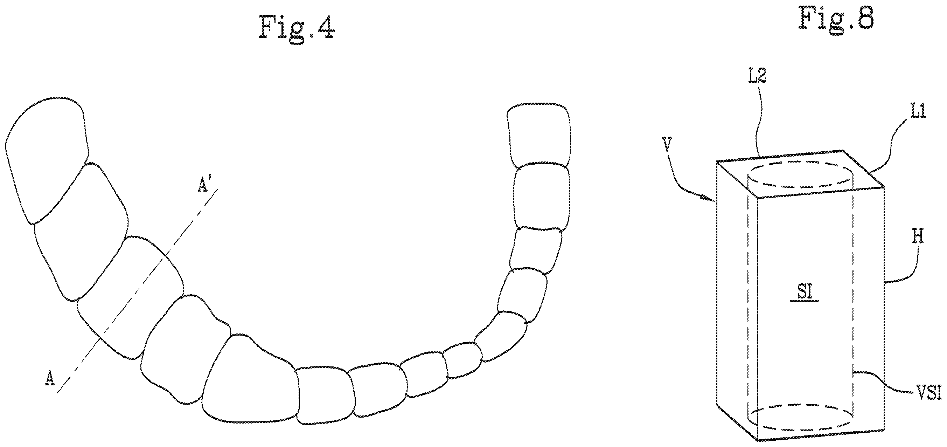

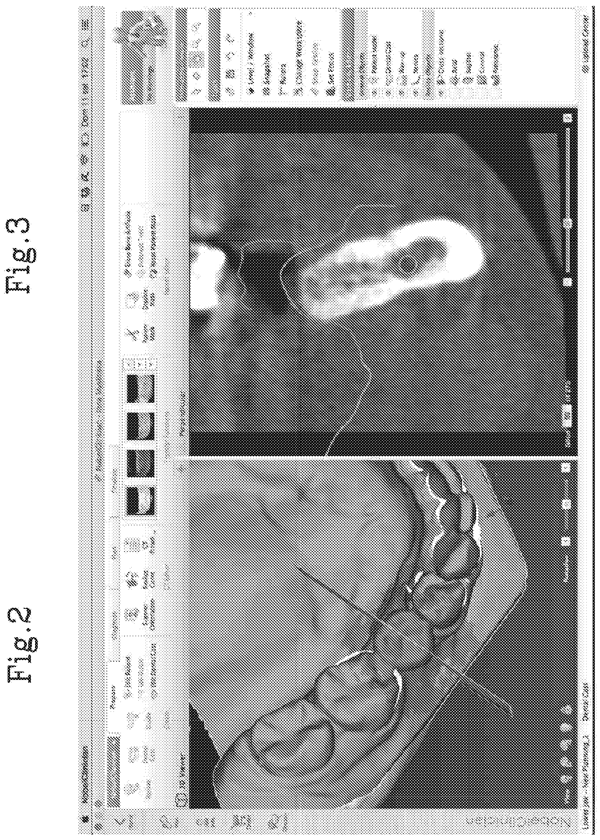

In particular, Figs. 2 and 4 respectively show a software simulation screenshotin plan view and a corresponding schematic view used for the present description.

More specifically, Figure 4 is a view of a portion of the maxillary arch of a patient, wherein a missing premolar tooth has been graphically simulated, and wherein an axis A-A' is represented inthe simulated premolar, which is adapted to ideally dissect it transversely with respect to the mandibular bone.

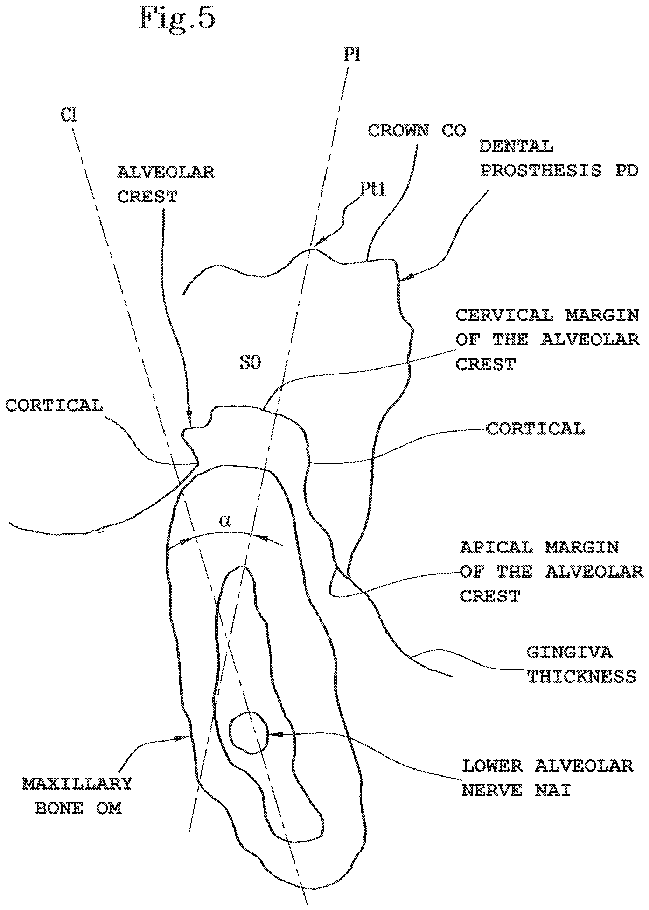

In particular, Figure 3 depicts one of a software simulation screenshot in sectional view, whereas Figure 5 shows a corresponding schematic view used for the present description.

In particular, Figure 5 is a sectional view along the axis A-A 'of Figure 4 of a mandibular bone and the simulated dental prosthesis, wherein there are shown a surgical ideal axis CI and an ideal prosthetic axis PI.

The graphic simulation step f1 according to the invention, allows to graphically simulate an anatomy of a provided dental prosthesis PD (fig. 5) and a respective edentulous site LR (fig.1) corresponding to an identified tooth Tn, wherein the provided dental prosthesis PD is coupled to a dental implant ID (fig.1).

More generally, the invention is applicable both in the case in which the Tn tooth is missing in the edentulous site, as well as in the case in which the Tn tooth is present in the edentulous site and needs to be rehabilitated. As shown in Figure 1, the ID dental implant is installed in a maxillary boneOM at the base of the edentulous site LR, in a position occupied by the identified tooth Tn, wherein Tn is the tooth identified in conditions of integrity of the patient's mouth.

Preferably, the graphic simulation step f1 is performed by way of a computed tomography (TAC) of the oral cavity that provides data based on which a 3D reconstruction of the maxllary bones may be effected. Alternatively or in addition, the graphic simulation is achieved by means of a conventional dental prosthesis model, which may also be obtained by intra or extra-oral scanning or virtually modeled.

An integrated graphical simulation is achieved by combiningthe intra or extra-oral scanning, or virtually derived scanning, with the 3D reconstruction made via the computed tomography.

Worded differently and to resume, the graphic simulation step f1 is carried out via computer-implemented graphic simulation means, in particular via a computer-implemented graphic simulation apparatus.

All processes performed are feasible starting from the first simulation step f1, without which an accurate detection of the oral cavity state would not be possible; non-automated detections, based essentially on the experience of the clinician, are totally inadequate for the subsequent simulation steps and can in no way lead to obtaining an *optimized* implant site.

In other words, the first simulation step f1 defines a computer-implemented mathematical graphic model based on which it is possible to perform innovative processing of the invention in order to obtain an accurate and optimized simulation in terms of implementation of an implant site; the implant site thus obtained will be configured for being coupled with a corresponding dental implant.In a particular example, based on the graphic simulation of Fig. 2 and the corresponding schematic view of fig. 4, a premolar tooth that will be added to the patient is shown sectioned by an A-A 'axis.

In particular, from the graphic simulation of Fig. 3 and the corresponding schematic view of fig. 5, there is shown the crown CO of the dental prosthesis PD and the maxillary bone OM which tends to white color where the bone is cortical, while tending to gray color where the bone is spongy; in the lower part of the maxillary bone, the lower maxillary nerve NAI is also present.

Starting from the data obtained from the simulation, the inventive method allows an assessment of the actual amount of themaxillary bone OM present at the edentulous site LR.

The technical effect achieved, is an exact structuring and sizing of the implant site Slsuch that the same may receive the dental implant ID thereby ensuring stability thereof.

The method of the invention provides to perform a calculation step f2 of a prosthetic axis PI ideal for the dental prostheses PD.

The ideal prosthetic axis is an axis passing through an ideal point of the dental prosthesis PDprovided, depending on the type of toothwhich is being treated.

In particular, in a first case of molars and premolars (fig.5), the ideal prosthetic axis PI for the intended dental prosthesis PD, is formed as an axis passing through the center pt1 of the intendeddental prosthesis PD.

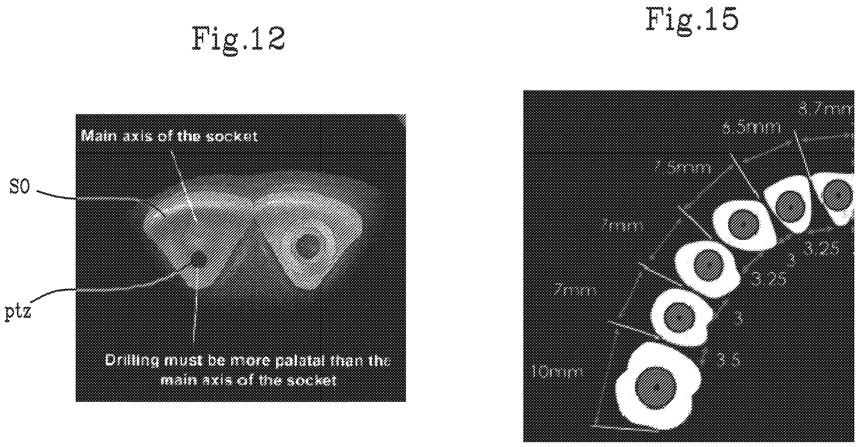

In particular, in a second case of lateral incisors and canines (fig.12), the ideal prosthetic axis PI for the intendeddental prosthesis PD, is formed as an axis passing through the palatal vertex pt2 of a triangle that schematizes the occlusal surface of the intendeddental prosthesis PD.

In other words, in a first case of molars and premolars, the ideal point pt1 will be at the center of the dental prosthesis PD and CO crown, whereas,in a second case of anterior teeth (incisor, lateral and canine), the ideal point pt2 will be at the apex of thepalatal angle.

In both cases, for the calculation of that axis, the method provides obtaining two pairs of measures of the simulated dental prosthesis PD, in particular a first pair of cervical measures and a second pair of apical measures which are defined in terms of statistical data dependent on the position of the tooth and the anatomy of the arch opposed to that into which the dental prosthesis PD is inserted.

The pair of measures is calculated in the the vestibule-lingual and anterior-posterior direction.

Precisely, the first pair of measures is taken at the crown of the dental prosthesis PD and in the vestibule-lingual and anterior-posterior direction. The second pair of measures is taken at the collar of the dental prosthesis PD and in the vestibule-lingual and anterior-posterior direction. Alternatively or in addition, the pair of measures is calculated in the mesiodistal direction.

To be precise, the first pair of measures is taken at the dental prosthesis crown PD, while the second pair of measures is taken at the level of the collar of the dental prosthesis PD. The method provides calculating the prosthetic axis PI as a straight line passing through the intersection points of the segments of each measurespair.

Since the OM maxillary bone exhibits a conformation which slightly degrades from the vestibule toward the tongue, it is requested to find the correct bone angle with respect to the tooth.

To this end, the method according to the invention provides for the calculation of two axes: the already described ideal prosthetic axis PI and an ideal surgical axis CI, which allows an ideal halving of the maxillary bone OM amountat the edentulous site LR.

In particular, as already mentioned, wherea PD dental prosthesis is applied to the mandibular arch, the bone OM is disposed below the prosthesis, whilst in the case of a PD dental prosthesis applied to the upper arch, the bone OM is disposed above the prosthesis.

In order to accuratelydeterminethe ideal surgical axis CI, the method of the invention provides a plurality of detections being representative values of the maxillary bone OM.

The technical effect achieved leads to a precise sizing and shape detectionof the bone,being necessary for obtaining a precise simulation of an implant site SI that is to be afforded within the bone itself.

In fig. 3 a dental prosthesis PD with underlying mandibular bone OM thereofis depicted in a sectional view along an A-A' plane transverse to the maxillary bone OM.

With particular reference to this figure and to Figure 1, the method according to the invention provides a calculation step f3 of a first cervical distance CT and a first apical distance ET of the maxillary bone OM relative to, and more particularly, below, the edentulous site LR.

According to the invention, the calculation of these distances is performedas a function of the graphic simulation of the stepf2 and in a first direction with respect to the maxillary bone OM.

Preferably, the first direction is substantially transverse with respect to the maxillary bone OM.

Preferably, the first cervical distance CT is a distance on the Vestibule-Lingual or Vestibule-Palatal plane between the two cortical at the height of the cervical margin of the maxillary crest.

In particular, in the case of very thin ridges it is considered a minimum width of at least 4 mm.

Preferably, the first apical ET distance is a distance on the Vestibule-Lingual or Vestibule-Palatal plane between the two cortical at the height of the apical margin.

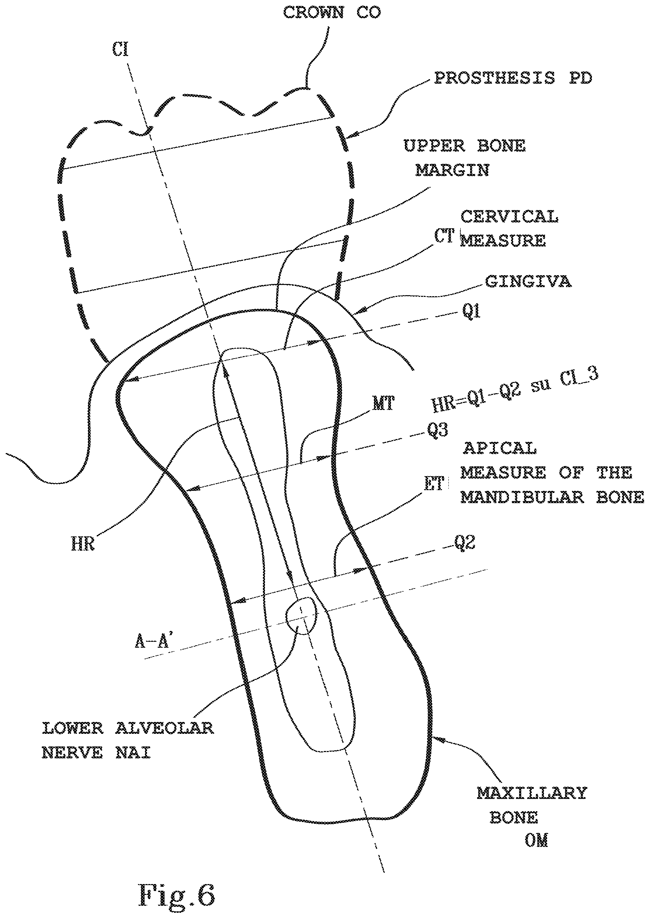

According to the invention, the first cervical distance CT is measured at a first cervical portion q1 with respect to a reference portion, for example with respect to an upper edge of the bone shown in Figure 6.

Preferably, in the latter case depicted in Fig. 6, the first cervical portion q1 is identified in reference to the upper margin of the bone crest where the latter is equal to or greater than 4 mm.

According to the invention, the first apical distance ET is detected at a first apical portion q2 with respect to a reference portion, in particular with respect to the lower maxillary nerve NAI in the posterior mandible, or to any other anatomical structure to be observed.

Preferably, in the case shown in Fig. 6, the first apical portion q2 is identified above the lower maxillary nerve NAI, preferably at a distance of 2 mm.

Alternatively, absent a nerve, the first apical distance ET is detected at a first apical portion q2 with respect to a reference portion, in particular it deals with an anatomical limit comprised between:

- the lower edge of the mandible (frontally to the NAI for lower arch) and the NAI posteriorly;
- maxillary sinus and nasal cavity (for upper arch);
- impacted teeth;
- cysts;
- anatomical defects.

The method according to the invention provides a calculation step f4 of a second cervical distance CW and a second apical distance EW of the maxillary bone OM at, and in particular below the edentulous site LR.

According to the invention, the calculation of these distances is made as a function of the stepf1 in a second direction with respect to the maxillary bone OM.

Preferably, the second direction is substantially longitudinal to themaxillary bone OM.

In particular, the second direction is perpendicular to the direction along which the distances CT and ET are calculated.

Preferably, the second cervical distance CW is the distance with respect to the bone crest between the limits of the edentulous site LR.

In other words, CW is the distance between the roots of the teeth adjacent to the edentulous site LR, where adjacent teeth exist, as shown in the case depicted in figure 1.

Absent the adjacent teeth, the CW calculation is made considering as if the teeth exist, i.e. considering as extremes the continuation into the bone of the planestangent to the mesial and distal sides of the tooth and perpendicular to the occlusal plane, as shown in Figure 15.

Alternatively, CW is the distance between a root of an adjacent tooth and another implant or anatomical limit.

According to the invention, the second cervical distance CW is detected at the same first cervical portion q1 used for the calculation of CT. According to the invention, the second apical distance EW is detected at the same first apical portion q2 used for the calculation of ET.

In other words, CT and CW are measured at the same portion.

The technical effect achieved is a schematic regular representation of the irregular anatomy.

The same reasoning applies to ET, EW MT and MW.

The overall technical effect is that of predisposing a regular volume within an irregular anatomy.

According to the invention, the method comprises the step f5 of calculatingan ideal surgical axis ($CI\_i$: i = 1..n) on the maxillary bone OM as a function of cervical distances CT, CW and apical distances CW, EW. In particular, according to the invention, the surgical axis CI is defined as an axis passing through two points, the first Cx obtained as anintersection between the representative segments of the cervical distances CT, CW, and the second Ex obtained as an intersection between the segments representative of the apical distances ET, EW.

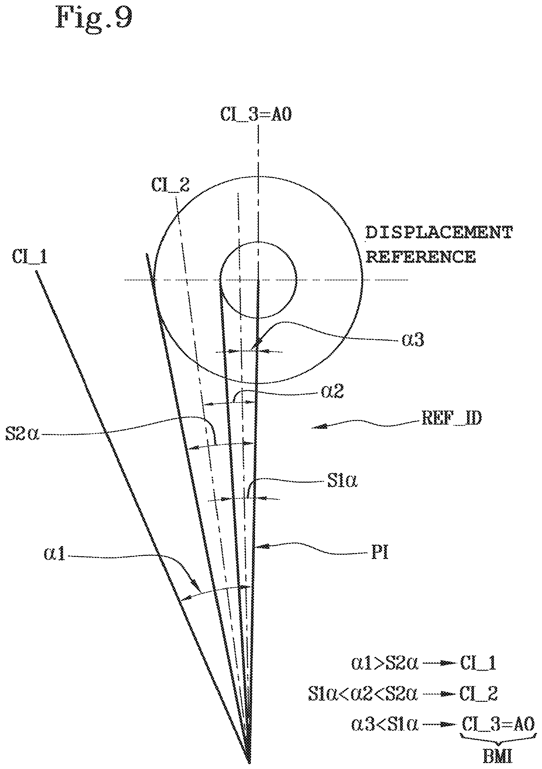

The surgical axis is calculated in this way both in the case of molars and premolars, as well as in the case of front teeth (incisor, lateral and canine). According to the invention, the set of references comprising the ideal prosthetic axis PI and the ideal surgical axis CI determines a reference system REF_ID (fig. 9) to the dental implant ID, wherein the ideal prosthetic axis PI and the ideal surgical axis CI are offset by a deviation angle $\alpha$. (Figure 5).

Figure 4 shows a schematic view of the first and second cervical distances CT, CW, and the first and second apical distances ET, EW and the ideal surgical axis CI obtained as previously described.

It is noted from the figure that the y directions along which first distances CT and ET are calculated, are substantially perpendicular to the x directions along which the seconddistances CW and EW are calculated. The calculated distances are representative of maxillary bone OM volume at the edentulous site LR.

The technical effect achieved by the identification of these distances is an identification of the overall dimensions of the maxillary bone OMintended to receive the implant site; the greater the overall dimensions, the greater the maximum diameter of a potential implant insertable into the maxillary bone.

In addition to the steps described above, the method according to the invention provides a calculation step f14 of a first median distance MT measured at a first median portion q3 (fig. 6) in the maxillary bone OM between the first cervical portion q1 relative to the first cervical distance CT, and the first apical portion q2 relative to the first apical distance ET. Preferably, said first median distance MT is at a median portion q3 that is intermediate between the first cervical portion q1 and the first apical portion q2.

The technical effect achieved by this further detection is an identification of the exact shape of the maxillary bone OM that allows detection of malformations (eg. cavity) in the bone that make the bone unsuitable for housing the implant site.

Alternatively or in addition to the calculation of the first median distance MT, the method according to the invention comprises a step f15 of calculating a second median distance MW measured at the same median portion q3 used for the calculation of MT.

The *technical effect* achieved by this additional measurement is an identification of the exact shape of the maxillary bone OM that allows detection of malformations (eg. cavity) in the bone that make the bone unsuitable for housing the implant site.

The calculation of the first median distance MT and of the second median distance MW is made by the fifth calculation module 105.

A further technical effect is achieved when the measurement of the second median distance MW is associated with the measurement of the first median distance MT; in this case the shape of the maxillary bone OM is reconstructed in an even more exactmanner, thereby allowing an optimal definition of the overall dimensionof the maxillary bone within which the implant site is being obtained.

In other words, the median distances MT, MW serve to evaluate the possible presence of a severe anatomical effect, (eg. a bone lack) which may heavily affect the dental implant diameter.

The inventive method further comprises the step f6 of calculating a bone height $HR\_i$ of the maxillary bone OM as a function of the calculated first cervical distance CT and first apical distance ET, as will be described in a later section.

The bone height HR is representative of a bone availability of the maxillary bone OM at the edentulous site LR, particularly therebelow.

According to the invention, the bone height HR is obtained as the difference on the surgical axis $CI\_i$ between the first cervical portion q1 associated with the cervical distance CT, and the first apical portion q2, associated with the apical distance ET.

A fifth calculation module 105 of the processing unit 10, described below, is configured to calculate the bone height HR.

The invention further comprises the step f7 of moving the ideal surgical axis $CI\_i$ relative to the ideal prosthetic axis PI maintained in a fixed position, thereby resulting in a compromise surgical axis $CI\_i$ (i = 1..n) corresponding to a deviation angle $\alpha i$ (i = 1..n) (fig.9) being variable with respect to the ideal prosthetic axis PI.

In other words, the method provides moving the ideal surgical axis CI by determining a compromise surgical axis $CI\_i$ (i = 1..n) corresponding to a deviation angle $\alpha i$ (i = 1..n) being variable with respect to the ideal prosthetic axis PI.

In other words, the invention method provides for calculating a deviation angle $\alpha i$ (i = 1..n) variable between a movable compromise surgical axis CI with (i = 1..n), and the ideal prosthetic axis (PI);

In yet other words, with reference to Figure 9, the invention provides moving the surgical ideal axis $CI\_1$ (which subtends a deviation angle $\alpha 1$), towards the ideal prosthetic axis PI maintained in a fixed position, thereby determining a compromise surgical axis $CI\_2$ that subtends a deviation angle $\alpha 2$.

The deviation angle $\alpha i$ (i = 1..n) is a representative measure of the maxillary bone OM inclination with respect to the dental prosthesis PD provided (i.e. with respect to the ideal prosthetic axis PI thereof); the more the maxillary bone is inclined with respect to the dental prosthesis PD, the more the ideal surgical axis is prevented from passing through the profile of the dental prosthesis, which means that it does not pass through the occlusal surface SO of the tooth.

The ideal surgical axis $CI\_i$ is moved so as to fall within the profile of the dental prosthesis PD; in fig. 9 the passing from the surgical axis $CI\_1$ to the surgical axis $CI\_2$, with corresponding passing of the deviation angle varying from $\alpha 1$ to $\alpha 2$, determines a surgical compromise axis which falls within the profile of the dental prosthesis PD.

According to the invention, the calculation of the bone height $HR\_i$ of the maxillary bone OM is made both as a function of the already calculated first cervical distance CT and first apical distance ET, and as a function of the variable deviation angle $\alpha i$ (i = 1..n ).

In other words, the more the ideal surgical prosthetic axis CI and the ideal axis PI are proximate to each other, the more the angle $\alpha$ decreases and the bone height value approaches an optimal value.

From a mathematical viewpoint, HR = (q1 - q2) / cos $\alpha$,

wherein $\alpha$ is = to the angle between the surgical axis CI and the prosthetic axis PI and wherein typically 0 ° < $\alpha$ < 90 °.

Since cos $\alpha$ < 1 between 0° and 90°, the bone height HR is always smaller than the difference between q1 and q2, except for the case where $\alpha$ = 0.

In other words, the more the ideal CI surgical axis approaches the prosthetic ideal axis PI, the more the angle $\alpha$ decreases, thus resulting in a bone height HR variation.

When determining the bone height HR, the invention provides for the following sizing:

- the first cervical distance CT must be greater than a first threshold distance $S1\_CT$ possibly adjustable, i.e. CT >= $S1\_CT$, wherein $S1\_CT$ is preferably = to 4 mm; in other words, $S1\_CT$ = 4 mm is the minimum value required for a dental implant ID being realized according to the invention without having to resort to a bone augmentation;
- the first apical distance ET must be greater than a second threshold distance $S2\_CT$, i.e. ET >= $S2\_CT$; in other words, $S2\_CT$ = 2 mm is taken at the positioned portion q2, preferably, at a distance of 2mm from a reference portion representative of an anatomical limit; preferably, the anatomical limit is the lower maxillary nerve NAI.

The technical effect achieved by the described sizing is to provide an implant site in an optimal position within the bone, so as to avoid any interference with proximal anatomical limits, thereby protecting the integrity of the patient as much as possible.

The invention provides that the values of the distances CT, ET, CW, EW may vary as a function of the variable deviation angle $\alpha i$ (i = 1..n).

In other words, the values pertaining to:

- the first cervical distance CT;
- the first apical distance ET;
- the second cervical distance CW;
- the second apical distance EW

are defined as a function of each identified single deviation angle $\alpha i$ (I = 1..n), thereby being corresponding values $CT\_i$, $ET\_i$, $CW\_i$, $EW\_i$ defined.

The invention provides a step f8 of simulating the optimized implant site SI for the dental implant ID in the maxillary bone OM at the edentulous site LR.

In one of the main aspects of the invention, which will be described in detail later, an implant site simulation system comprises a processing unit 10, in turn comprising a simulation module 107B configured to implement the simulation step f8.

The optimized implant site SI exhibits an at least partially cylindrical volumetric shape VSI inscribed within an expected circumscribing volume V.

According to the invention, the optimized implant site SI is simulated at least as a function of:

☐ the identified tooth Tn;

☐ a first cervical distance CT_i and a first apical distance ET_i;

☐ a second cervical distance CWi and a second apical distance EWI;

☐ the bone height HR_i;

☐ the variable deviation angle αi (i = 1..n).

In other words, a new implant site is calculated whenever there is a variation in the deviation angle αi, which in turn determines a variation in reference marginal and apical distances and in the bone height.

In one embodiment of the invention, the distances CTi, ETI, CWi, EWI are variable depending on the variable deviation angle αi, thereby determining an optimized implant site SI when the variable deviation angle is minimized.

The invention therefore provides for calculating implementation values of the optimized implant site SI as a function of the simulation, thereby determining an optimized implant site SI configured to receive the corresponding dental implant ID.

The 107B simulation module is configured in particular to calculate implementation values of the optimized implant site SI as a function of the simulation, thereby determining an optimized implant site configured to receive the corresponding dental implant ID.

In particular, the variable deviation angle is minimized when the compromise surgical axis CI_i tends to the ideal surgical axis Clas much as possible, thus ensuring a balance of forces on the PD prosthesis which ensures stability / feasibility thereof.

In a third aspect, the invention comprises, in the first instance, an optimized implant site SI obtained with the method described up to now.

In a fourth aspect, the invention comprises, in the first instance, a dental implant ID which may be coupled to the optimized implant site SI.

The invention further provides a step f12 of comparing the variable deviation angle αi (i = 1..n) with the predefined threshold values of the deviation angles S1α, S2α (fig.9).

The threshold values are representative of a surgical axis CI_i position with respect to an occlusal surface SO of the dental prosthesis PD.

In other words,

- the first deviation threshold value S1α is the greaterlimit angle of the deviation angle αi (i = 1..n) for a definition of an optimized dental implant (Best Matchinglmplant BMI) otherwise defined as limit inclination angle of the ideal surgical axis CI_i,based on which the angle between the ideal prosthetic axis PI and the ideal CI surgical axis may be considered as minimized.
- the second deviation threshold value S2α is the greaterlimit angle of the deviation angle αi (i = 1..ne S2α> S1α) such that the ideal surgical axis CI_i passes through the profile of the dental prosthesis PD, that is, passes through the occlusal surface SO of the tooth.

Preferably, the invention provides that the comparison step f10 comprises the steps of:

(f6) recalculating bone thickness HR_i;
(f7) moving the ideal surgical axis CI_i relative to the ideal prosthetic axis PI_i maintained in a fixed position, thereby resulting in a variation of the variable deviation angle αi (i = 1..n);
according to the invention, the steps herein described are performed until the variable deviation angle αi remains incompatible with the predefined deviation angles threshold values of the S1α, S2α.

In other words, the steps f6 and f7 are repeated until the condition remains based on which the deviation angle αi is greater than the first deviation threshold value S1α, i.e. αi> S1α).

The deviation angle αi (i = 1..n) is minimized when is smaller than the first predefined deviation angle threshold values S1α.

In other words, when CI_i varies its angle αi relative to the prosthetic axis PI, the distances CT, ET, MT consequentlyvary the measure thereof,in thatthe angle varies based on which the segments CT, MT, ET are crossing transversely the maxillary bone OM (i.e. from tongue to vestibule).

The invention provides, at this stage, the step f10 of identifying at least one optimized dental implant within the implant site SI_I as a function of a variable deviation angle αi (i = 1..n), compatible with the predefined deviation angle threshold values S1α; S2α.

The values S1α, S2α are representative of thepassing of theideal surgical axis CI_i through a portion of the dental prosthesis PD of said occlusal surface SO.

In summary:

$$S2\alpha < \alpha i$$

If CI_i and PI are offset by a variable deviation angle αi which is greater than the second predefinedthreshold value S2α, there are generated moments of forces on the prosthesis PD which is to be inserted into the edentulous LR site that may cause a displacement ofthe PD prosthesis resulting in the prosthesis escaping from the edentulous site LR.

$$S1\alpha < \alpha i < S2\alpha$$

Conversely, when S1$\alpha$ <$\alpha i$ <S2$\alpha$, while not beingoptimal, the implant positionis not even unacceptable since the moments of force acting on the tooth are minimal, thereby remaining the PD prosthesis substantially stable.

$$\alpha i < S1\alpha$$

If CI_i and PI are offset by $\alpha i$ <S1$\alpha$, the value $\alpha i$ <S1$\alpha$ determines the optimaloffset angle $\alpha$OTT between CI and PI.

The calculation of the optimal implant site SI takes place at the optimal offset value $\alpha i$.

In the third aspect of the invention, the implant site SI is optimized according to the invention and is representative of the best compromise for the inclination angle of the implant between the extreme positions of the ideal surgical axis CI and the ideal prosthetic axis PI.

According to the invention, the optimized implant site SI achieves the technical effect of enabling the dental implant ID installed therein, to avoid arising ofmechanical moments of forces which would result beingunfavorable to the tooth permanence in the edentulous site LR.

In fact, because during mastication the masticatory force is along the PI axis, which means that the stress arising therefrom is a compressive stress and not a flexural stress, from a mechanical viewpoint, the PD dental prosthesis should lie on saidprosthetic axis PI.

Where the deviation angle between the surgical axis and the prosthetic axis is minimized, there are no moments of force being able to "destabilize" the dental prosthesis, which means that the arm that is created between the ideal surgical axis CI and the prosthetic axis PI has substantially null values. In such acase, the implant site would be suitable to accommodate animplant considered to be optimized.

With reference to Figure 9, a case of optimal surgical axis is indicated which is referred to with CI_3.

In the case where the surgical axis is offset with respect to the prosthetic axis such that the surgical axis is not crossing the profile of the dental prosthesis PD, i.e.it is not passing through the occlusal surface of the tooth, a couple of forces is generated due to the fact that the arm between CI and PI is not null and the resulting implant is not optimal.

With reference to Figure 9, a case of non-optimal surgical axis is indicated by CI_1.

In the fourth aspect of the invention, a dental implant ID may be coupled to the optimized implant site SI obtained as already described.

The invention provides simulating the optimized implant site SI (stepf8), in which the optimized implant site SI exhibits anat least partially cylindrical volumetric shape VSI inscribed within the circumscribing volume V.

The simulation module 107B is configured to simulate the optimized SI implant site in the maxillary bone OM, in which the optimized implant site SI has at least a partially cylindrical volumetric shape VSI inscribed within an envisaged circumscribing volume V.

In particular, the optimized implant site has a cylindrical shape.

With particular reference to Figures 1 and 8, the implant site SI according to the invention, may be calculated as a function of an expected circumscribing volume V having a first dimension L1, a second dimension L2 and a third dimension H.

Preferably, the circumscribing volume V is shaped in a first approximation as a parallelepiped.

According to the invention, the first dimension L1 is calculated as a function of at least the first cervical distance CT and the first apical distance ET.

Preferably, the first L1 size is defined as the minimum measure between the first cervical distance CT and the first apical distance ET decreased by a predefinable safety margin $\Delta$L1.

In other words, L1 = min (CT, ET) -$\Delta$L1

In general terms, the value of the first dimension L1 is selectable by the clinician.

In particular $\Delta$L1 = 3mm. According to the invention, the second dimension L2 is calculated as a function at least of the second cervical distance CW and second apical distance EW.

Preferably, the second dimension L2 is defined as the minimum measure between the second cervical distance CW and the second apical distance EW decreased by a predefinable safety margin $\Delta$L2.

In other words, L2 = min (CW, EW) -$\Delta$L2.

Generally speaking, the value of the first dimension L2 is selectable by the clinician.

In particular $\Delta$L2 = 3mm.

According to the invention, the third dimension L3 is calculated as a function at least of the bone height HR.

In particular, in the example described in the figures, the first dimension L1 is representative of a buccal-lingual distance, the second dimension L2 is representative of a mesial-distal distance,whereas the third L3 dimension is representative of a depth of the optimized implant site at the edentulous site LR, particularlyat an underlying zone.

In other words, the implant site SI according to the invention is obtainable as cylinder inscribed in the circumscribing volume V.

According to the invention, the calculation of the implant site SI starting from an expected circumscribing volume V, is also made as a function of the first median distance MT and / or the second median distance MW.

In other words, according to the invention, the first dimension L1 is calculated as a function of the first cervical distance CT, the first apical distance ET and the first median distance MT.

According to the invention, the second dimension L2 is calculated as a function of the second cervical distance CW of the second apical distance EW and the second median distance MW.

The technical effect achieved is the simulation of an implant site entirelycompatible with the dimensional and structural characteristics of the receiving maxillary bone OM.

Once the optimized implant site SI has been simulated, the method provides several evaluations based onthe parameters being representative of the dental implant ID, which dental implant ID may be coupled to the optimized implant site after calculating the new HR_i and resulting new values of CTi ETI, CWi, EWI, HRi, αi, on the basis of the new CI_2 axis.

A first parameter to be evaluated is a dental implant (ID) head (TI) position.

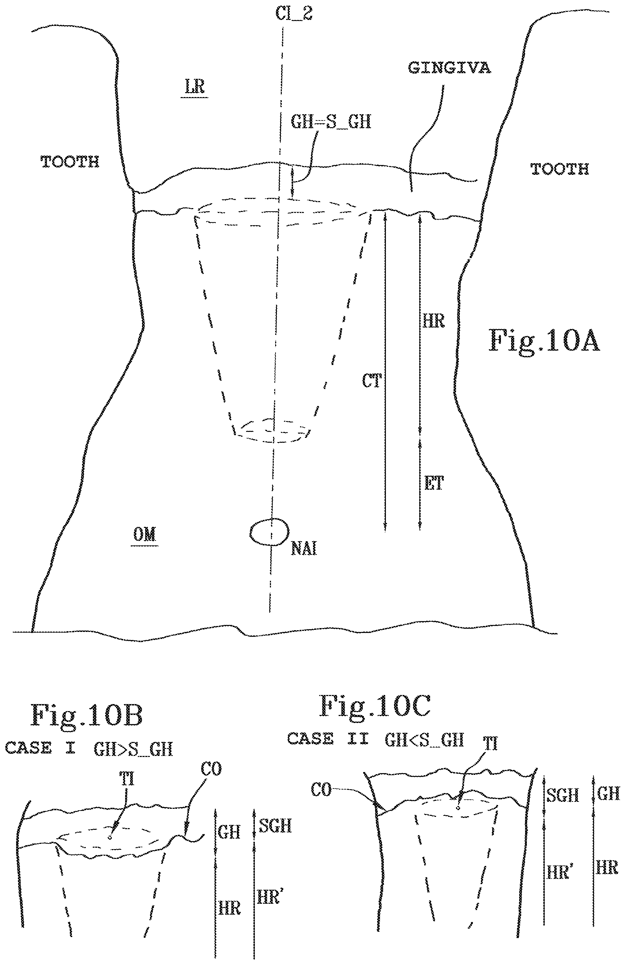

With reference to the group of Figures 10A, 10B and 10C according to the invention, the calculation of the head TI position is made as a function of the gingival height GH being indicative of the gingival thickness above the bone crest CO of the maxillary bone OM along the concernedsurgical axis. The head position TI calculation is further madeas a function of a predefinable threshold gingival height S_GH,which is also defined as the distance between the maxillary bone OM and the lower part of the dental prosthesis PD.

The simulation module 107B is configured to perform calculation of the position of the head TI.

According to the invention, the step of evaluating the position of the head (TI) of the dental implant (ID) comprises the substeps of:

- where the gingival height GH is >than the predefined threshold gingival height S_GH(see Fig. 10B), increasing the bone thickness HR_i with following increase in the first cervical distance CT, or increasingin the height of the crown CO obtainedby fixing the position value of the head TI at the value of the first cervical distance CT.
- where the gingival height GH is<than the predefined threshold gingival height S_GH (see Fig. 10C),then calculating a new bone thickness value HR_i and assigning to the first cervical distance CT the new bone thickness value HR_i; in other words, the new bone thickness value HR_i is such that HR_i = HR + GH - S_GH.
- where the gingival height GH is = to the predefined threshold gingival height S_GH(see Figs. 10A and 9 with surgical axis CI_3),considering the current bone thickness HR_i as the optimal representative thickness of the correct position of the head TI and storing the value of first current cervical distance CT.

Preferably, 3mm <S_GH <4 mm.

More preferably, S_GH = 3.5 mm.

Consequently the length of the implant site LI is defined as a function of the bone height calculated HR_i.

The simulation module 107B is configured to simulate the implant site SI as a function of the implant site length LI defined as a function of the optimal bone height HR-i.

A second parameter to be evaluated is a measure of a diameter DT of the dental implant head TI.

The measure of the DT diameter is calculated as a function of a minimum measure between the distances CT, MT, ET and CW, MW, EW, as a function of a first predefined threshold diameter value S_D1.

The simulation module 107B is configured for this calculation..

The method of the invention provides namely the step of determining a measure of a DT diameter of the implant head TI as comprising the substeps of:

- calculating the minimum measure MIN_L between the distances CT, MT, ET and CW, MW, EW;
- calculating the diameter DT as a function of the minimum calculated measure MIN_L and a first predefinedthreshold diameter value S_D1. The technical effect is achieved based on which the head TI is uncapable of breaking through the buccal peak or lingual peak.

In other words, the method allows to determine the diameter of the implant head with the certainty that the implant will definitely be comprised withinthe maxillary bone, namely the head thereof does not "brake through" the buccal or lingual peak.

In most cases the minimum measure MIN_L is the first cervical distance CT.

Preferably, the diameter DT is calculated as the difference between the calculated minimum measure MIN_L and the first predefinedthreshold diameter value S_D1.

Preferably, such a minimum measure being MIN_L = 3mm.

Preferably the diameter threshold value S_D1 is defined according to the "type" of tooth the implant which is being realized is intended for.

These values are defined in the literature,e.g.S_D1> = 4.5 in respect to molars and S_D1> = 3.5 in respect to premolars.

It follows that the optimized implant site SI is implemented as a function of the diameter DT,obtained as the diameter of the cylinder inscribed in the circumscribing volume V, wherein the diameter DT is calculated as a function of the first dimension L1 and second dimension L2.

In particular, the simulation module 107B is configured to simulate the optimized implant site SI according to the aforementioned diameter DT. The method provides for an evaluation of the compatibility of the diameter measure DT with predefined bone thickness thresholds, in particular a first thickness threshold S_VE calculated with respect to the vestibular peak PV, and a second thickness threshold S_PL calculated with respect to the lingual peak PL.

The method particularly provides to determine an optimal implantwhen the following occurs:

- the diameter DT is at a distance> = to the first thickness threshold S_VE compared to the vestibular peak PV;
- the diameter DT is at a distance> = to the second threshold S_PL thickness compared to the lingual peak PL.

In one preferred embodiment of the invention, the predefineddiameter threshold value S_DI is obtained as the sum of the first thickness threshold S_VE and the second threshold thickness S_PL.

Preferably S_VE = 2mm
Preferably S_PL = 1mm

The technical effect achieved is that the implant will definitely be comprised withinthe maxillary bone, substantially centered between the vestibular peak and lingual peak.

A third parameter to be evaluated is a measure of an implant LI length of the dental implant ID.

According to the invention, the step of determining the implant LI length is implemented as a function of a recalculated value of the bone thickness HR_i.

A fourth parameter to be evaluated is a measure of a crown/root RL/RCratio.

A fifth parameter to be evaluated is animplant percentage in the bone PO. A sixth parameter to be evaluated is a bone density DO, defined as a function of a preset density value HF.

The optimization degree of the implant site SI is evaluated based on a rating (i.e. an evaluation) being assigned to the values of the parameters representative of the simulated implant site.

The technical effect achieved is the simulation of an optimal implant site SIto which a plurality of available implantscan be compared including selecting the best existing dental implant IE.

The simulation of the optimal implant site SI is implemented as a function of a global rating R of theoptimized implant site SI defined as a function of one or more of:

- a rating of a deviation angle R_$\alpha$i of said deviation angle $\alpha$i;
- a diameter rating R_DT of the diameter DT;
- alength rating R_LI of the lenght measureLI of the optimized implant site SI;
- a crown / root R_RL_RC ratio rating;
- a percentage rating of the implantin the bone R_PO;
- a bone density rating R_DO,

wherein the rating values Rai, GDR, RLI, RRLRC, RPO, RDO of theoptimized implant site SI are defined as a function of a comparison between the current values $\alpha$i, DT, LI, RLRC, PO, DO and respective reference thresholds below described.

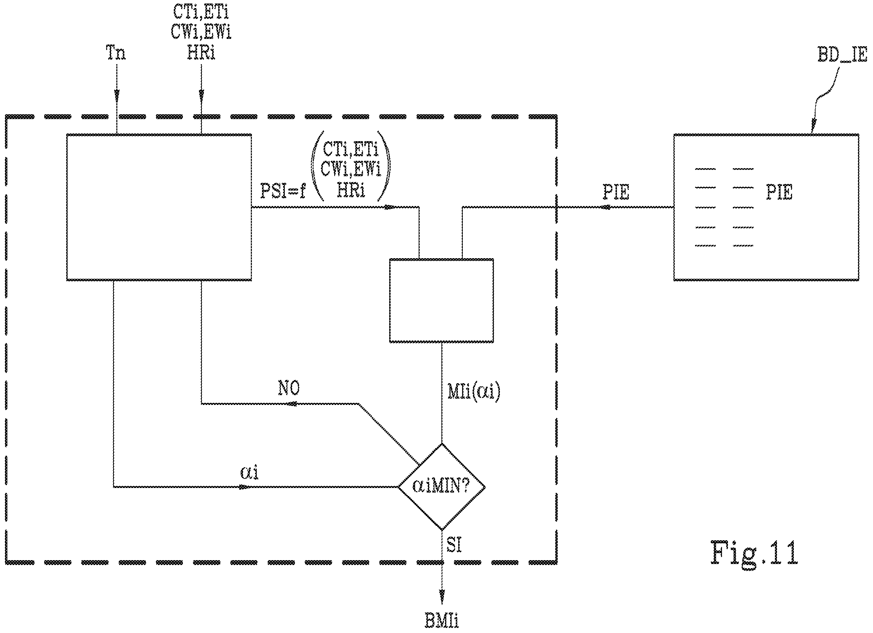

In one embodiment, the invention provides the step 9 of preparing a database BD_IE (fig. 11) comprising second representative parameters PIE of existing dental implants IE.

The IE existing dental implants are preferably cylinder-shaped,whilethe second representative parameters PIE include diameter and length of the cylinder.

Typically the database BD_IE,which possibly may be consulted remotely, includes the second representative parameters PIE being representative of:

- a tooth identifier (ETN) for an existing implant (IE);
- an implant diameter (EDTI) for an existing implant (IE);
- animplant height (ELIi) for an existing implant (IE);
- a deviation angle (Eai) for an existing implant (IE);
- a rating identifier (ERI) for an existing implant (IE);
- an implant manufacturer;
- an implant model.

In one embodiment, the optimized implant site SI is instead calculated at least as a function of its first representative parameters PSI comprising at least:

- a diameter DTi of the at least partially cylindrical volumetric shape VSI;
- an heightLIi of the at least partially cylindrical volumetric shape VSI;
- the variable deviation angle $\alpha$i.

The invention provides a step f10 of comparing the first representative parameters PSI with the second representative parameters PIE. Advantageously, according to the invention, there is provided a step f11 of identifying one or more dental implants MII for the implant site SI optimized as a function of the comparison between the first representative parameters PSI and the second representative parameters PIE.

In step f8 simulating the optimized implantsite SI, the first representative parameters PSI further comprise one or more rating values (Rai, GDR, RLI, RRLRC, RPO, RDO) of the optimized implant site SI, defined as a function of a comparison among the first representative parameters PSI and the reference threshold values, which will be described hereinafter. The optimized implant site SI is identified with an R rating defined in acombined function of the rating values Rai, GDR, RLI, RRLRC, RPO, RDO.

In one embodiment, the implant site SI is optimized when the R rating is minimized.

According to the invention, the step f11 identifies one or more optimal dental implants BMII as a function of the comparison between the first representative parameters PSI and the second representative parameters PIE when the rating R is minimized.

As will be described in more detail below, the identification system of the invention comprises a second processing unit 20 comprising a comparison module 201 configured to compare the first representative parameters PSI with the second representative parameters PIE.

According to the invention, the comparison between the first representative parameters PSI and second representative parameters PIE is made as a function of a different comparison priority Pconf.

In other words, certain parameters such as the deviation angle, have a higher incidence on the rating than others.

According to the invention, the comparison between the optimized simulated implant site SI and the existing available dental implants IE, is made as a function of the technical representative parameters of the couplable implant site/dental implant, and the rating values associated with the comparison between these parameters and the corresponding actual values of existing available implants EI.

In particular, according to the invention, there is provided the step f13 of assigning a rating R_$\alpha$i to the dental implant ID as a function of the comparison between the variable deviation angle $\alpha$i (i = 1..n) and the predefined deviation angle threshold values S1$\alpha$, S2$\alpha$, analyzed in the comparing step f12.

For this purpose, as will be described in more detail below, the second processing unit 20 comprises an optimization module 204 configured to perform all the steps of rating assignment to the implant site.

The optimal implant BMII according to the invention is the best compromise for the implant deviation angle $\alpha$i between the extreme positions of the ideal surgical axis CI and the ideal prosthetic axis PI. According to the invention, a rating is associated to each compromise position; based on the rating calculation dependent on such compromise and further functional dependencies that will be described later in this section, one can determine the optimal way to be adopted for obtaining the BMI.

In particular, upon shifting the surgical axis CI toward the prosthetic axis PI, hypothetical dental implants ID_i are identified, which are implementable with a different rating each.

More specifically,

- if $\alpha$i > S2$\alpha$ → the rating is = - 2: the score is unfavorable (case CI_1 of Figure 9);
- if S1$\alpha$ < $\alpha$i < S2$\alpha$ → the rating is = - 1 (case CI_2 of Figure 9);
- if $\alpha$i < S1$\alpha$ → the rating is = 0 → BMI (case CI_3 of Figure 9);

In other words, the step f13 of assigning a rating to the implant site Rai SI is defined as a function of the step f12 of comparing the variable deviation angle $\alpha$i (i = 1..n) with predefined deviation angle threshold values

S1$\alpha$, S2$\alpha$, wherein the rating Rai decreases upon decreasing of the deviation angle $\alpha$i, and tends to zero when the deviation angle tends to the optimized value thereof.

The invention provides that the step f13 of assigning a rating includes the steps of:

- (f6) moving the ideal surgical axis (CI_i);
- (f7) calculating a bone height (HR_i; i = 1..n) of said bone;
- (f8) recalculating the distances CT, ET, CW, EW as a function of the variable deviation angle $\alpha$i (i = 1..n);
- identifying a corresponding optimized implant site SI;
- identifying corresponding dental implants BMIi and corresponding variable rating value R.

In a particular embodiment, the step of assigning a rating comprises the steps of:
where the rating remains with a value other than,

- moving the surgical axis (CI, CI_i)
- calculating a bone height (HR_i; i = 1..n) of said bone;
- recalculating (CTi, ETI, CWi, EWI) as a function of the variable deviation angle ($\alpha$i; i = 1..n);
- identifying the corresponding optimal implant site SI;
- identifying the corresponding optimal dental implants BMI.

In summary, according to the invention, the surgical axis is first defined and subsequently moved progressively towards the prosthetic axis; for each iteration i (i = 1..n) from the initial position of the surgical axis CI_i (i = 1) up to the final position in which said ideal surgical axis CI_i is crossing the occlusal surface SO (e.g. Ci_3), the software defines configuration values CTi, ETI, CWi, EWI, HRi, $\alpha$i of a corresponding implant site/dental implant of which a rating is calculated.

In other words, to each implant site/dental implant defined at least by the configuration values CTi, ETI, CWi, EWI, HRi, $\alpha$i, a rating is associated by the evaluation software, and the implant with the best rating will then define the BMI.

To be noted is the importance of assigning a rating to the surgical axis in all simulated positions in that it is not obvious that the theoretically optimal position (i.e. the one in which the surgical axis CI is substantially coincident with the prosthetic axis PI), is reachable or achievable; it may occur in fact that the particular conformation/shape of the maxillary bone makes not practicable the theoretically optimal implants.

Advantageously, the invention software provides simulating a plurality of theoretically optimal and non optimal implants, as well as calculating a rating which allows to subsequently define the actual BMI - not necessarily coinciding with the theoretical optimum implant - as a function of the technical and structural characteristics of the bone.

The technical effect achieved is determining an optimal dental implant both as a function of a structural logical theoretical calculation and as a function of a calculation of the actual sizing of the technical-structural bone variables.

According to the invention, the first representative parameters PSI and second representative parameters PIE respectively comprise the diameter DTi of the at least partially cylindrical volumetric shape VSI, and the implant diameter EDTI for the existing implant IE, obtained with the substeps of:

- calculating a minimum measure MIN_L between the distances CT, MT, ET and CW, MW, EW;
- calculating the diameter DTi, EDTI as a function of the minimum measure MIN_L calculated, and a first predefined diameter threshold value S_D1;
- checking that DTi or EDTI is at a distance that is > = than a first thickness threshold S_VE compared to the vestibular peak PV;
- checking that DTi or EDTi is at a distance being > = than a second thickness threshold S_PL compared to the lingual peak PL.

The invention provides to assign a rating to the measure of a diameter DTi or EDTI wherein:

- if the diameter DTi or EDTI is < than the second predefined value of threshold diameter S_D2, then →the rating is = to -1;
- if the diameter DTi or EDTI is < than the third predefined value of threshold diameter S_D3, then →the rating is = to -2.

Preferably, the second predefined value of threshold diameter S_D2 is = to 0.2 mm for premolars.
Preferably, the third predefined value of threshold diameter S_D3 is = to 0.5 mm for premolars.
According to the invention, the first representative parameters PSI and the second representative parameters PIE respectively comprise the height LII of the at least partially cylindrical volumetric shape and the implant height ELIi for the existing implant IE, obtained as a function of a value of the bone height HR_i.
The invention provides to assign a rating to the height measure LII, ELIi as a function of a first predefined value of threshold height SL_1.
If LI is >than the first predefined value of threshold length (SL_1), then →the rating is = to -2
If LI is < than the first predefined value of the threshold length (SL_1), then →the rating is = to -1
Preferably, SL_1 is = to 2 mm
The preferred values are defined according to the type of tooth and the reference literature.
According to the invention, the first representative parameters PSI and the second representative parameters PIE include a crown / root ratio RL_RC. The invention provides to assign a rating R_RI_RC to the crown/root ratio RL_RC, wherein if RL / CL is > = with respect to a first threshold SRLRC1, (in particular = 1), then →the rating is = to 0

if RL / CL is <SRLRC1, then →the rating is -1;
if RL / CL is <SRLRC2 <SRLRC1, then→ the rating is -2.
Preferably, SRLRC1 = 1;
Preferably, SRLRC2 = 0.75.

In particular, if RL / CL is > = 1, then→the rating is = to 0; in other words, if the root length RL is >than the crown length CL, the rating is = to 0 in that the technical effect of a more stable tooth is attained.

If 0.75 is <RL / CL <1, then →the rating is -1; In other words, if the length of the root RL is < than the crown length CL, the tooth is more unstable;
if RL / CL is <0.75, then→ the rating is -2, thus resulting in the tooth to be even more unstable.

According to the invention, the first representative parameters PSI and the second representative parameters PIE comprise a implant percentage PO in the maxillary bone OM.
The invention provides to assign a rating R_PO to the implant percentage PO in the bone;

- if PO is = to 100%, then→ the rating is = to 0;
- if a first threshold PO1 is <PO < than a second threshold PO2, then→the rating is -1;

If PO is < than the first threshold PO1, then→the rating is -2.
Preferably, the first threshold PO1 is = to 95%.
Preferably, the second threshold PO2 is = to 99%.
According to the invention, the first representative parameters PSI and second representative parameters PIE include a bone density DO.
The invention provides to assign a rating R_DO to the bone density PO, wherein:

if DO is > than the threshold density SDO, then→the rating is = to 0
if I DO is< than the threshold density SDO, then →the rating is = to -1

According to the invention, the simulation module 107B is configured to simulate the optimized implant site SI as a function of one or more of;

- the crown / root ratio;
- the percentage of implant in the bone PO;
- the density DO of the maxillary bone OM;
- the global rating R of the optimized implant site SI.

In a second aspect, the present invention describes a computer implemented method.
In a third aspect, the present invention describes a **system of identifying** a dental implant for an implant site.

In a fourth aspect, the present invention describes a dental implant obtained by the method of the first aspect or the system of the third aspect of the invention as described in claim 25.

In a fifth aspect, the present invention describes a computer program configured to implement the method of the second aspect, as described in claim 22.

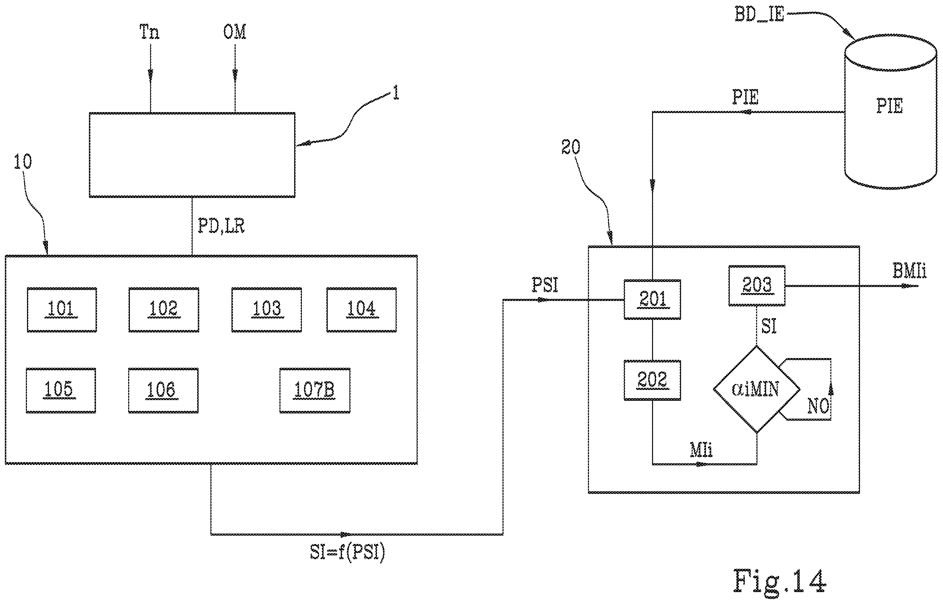

The system of identifying a dental implant for an implant site of the third aspect, is described in reference to Figure 14.

The system of identifying at least one dental implant MI for an implant site comprises:

Computer-implemented graphic simulation means 1 configured to graphically simulate an anatomy of a dental prosthesis PD providedand a respective edentulous site LR corresponding to an identified tooth Tn, wherein said dental prosthesis PD provided, is couplable to a dental implant ID insertable into a maxillary bone OM;
a processing unit 10 configured to simulate an implant site optimized SI starting from the simulation of the anatomy of the dental prosthesis PD provided, wherein the processing unit 10 comprises:

- simulation means 1 configured to graphically simulate an anatomy of a provided dental prosthesis PD and a respective edentulous site LR corresponding to an identified tooth Tn, wherein said provided dental prosthesis PD is couplable to a dental implant ID insertable into a maxillary bone OM;

a processing unit 10 configured to simulate an optimized implant site SI starting from the simulation of the anatomy of the provided dental prosthesis PD, wherein the processing unit 10 comprises:

- a first calculation module 101 configured to calculate a prosthetic axis PI for the providedideal dental prosthesis PD as a function of said simulation wherein the ideal prosthetic axis PI is an axis crossing an ideal point of the envisaged dental prosthesis PD according to the type of tooth Tn considered;
- a second calculation module 102 configured to calculate a first cervical distance CT and a first apical distance ET of said maxillary bone OM at said edentulous site LR as a function of said graphic simulation, wherein the distances are calculated in a first direction with respect to said maxillary boneOM.

Preferably, the first direction is substantially transverse to the maxillary bone OM.

- a third calculation module 103 configured to calculate a second cervical distance CW and a second apical distance EW of said maxillary boneOM at said edentulous site LR, as a function of said graphic simulation, wherein the distances are calculated in a second direction with resepct to the maxillary bone OM.

Preferably, the second direction is substantially longitudinal to the maxillary bone OM.

- a fourth calculation module 104 configured to calculate anideal surgical axis CI on said maxillary boneOM as a function of said cervical distances CT, CW and said apical distances CW, EW;

wherein said ideal surgical axis (CI) is an axis configured to perform an ideal halving of the bone quantity of the maxillary bone (OM) at the edentulous site (LR)
wherein the ideal prosthetic axis PI and the ideal surgical axis CI determine a reference system REF_ID for said dental implant ID;
wherein said ideal prosthetic axis (PI) and said ideal surgical axis CI are offset by a deviation angle $\alpha$;

- a fifth calculation module 105 configured to calculate a bone height HR of saidmaxillary bone OM as a function of said calculated first cervical distance CT and first apical distance ET, wherein said bone height HR is representative of a bone availability of said maxillary bone OM at said edentulous siteLR;
- a motion control module (106) configured to move said ideal surgical axis CI by determining a compromise surgical axis CI (i; i = 1..n) corresponding to a variable deviation angle $\alpha i$ (i = 1..n) compared to said ideal prosthetic axis PI;
- a simulation module 107B configured to simulate said optimized implant site SI in said maxillary boneOM, wherein said optimized implant site (SI) exhibits an at least partially cylindrical volumetric shape (VSI) inscribed within a provided circumscribing volume (V), said simulation being created at least as a function of:

□ said identified tooth Tn;

said first cervical distance CTi and a said first apical distance ETI;
said second cervical distance CWi and a said second apical distance EWI;
said first bone height HR;
said variable deviation angle $\alpha i$;

wherein the optimized implant site SI is also simulated as a function of the first representative parameters PSI thereof comprising at least:

a diameter DTi of said at least partially cylindrical volumetric shape VSI

a height Lli of said at least partially cylindrical volumetric shape VSI; wherein said first representative parameters (PSI) have variable values as a function of said variable deviation angle ($\alpha$i);

- a database BD-IE comprising second representative parameters PIE of existing dental implants IE;
a second processing unit 20 comprising:

  - a comparison module 201 configured to compare said first representative parameters PSI with said second representative parameters PIE;
  - a first identification module 202 configured to identify one or more dental implants MiI for said implant site SI as a function of the comparison made by the comparison module 201.

According to the invention, in the simulation module 107B, the first representative parameters PSI further comprise one or more rating values Rai, GDR, RLI, RRLRC, RPO, RDO of the optimized implant site SI, defined as a function of a comparison between the first representative parameters PSI and reference threshold values; wherein the implant site SI is identified with a rating R defined as a combined function of said rating values Rai, GDR, RLI, RRLRC, RPO, RDO.

According to the invention, the second processing unit 20 further comprises a second identification module 203 configured to identify one or more optimal dental implants BMIi as a function of the comparison performed by said comparison module 201 when the rating R is minimized.

The second processing unit 20 further comprises an optimization module 204 configured for: assigning a rating (Rai) to said implant site (SI) as a function of the comparison performed by the comparison module 201 (step (f12)) between said variable deviation angle ($\alpha$i; i = 1..n) and predefined threshold values of deviation angles (S1$\alpha$; S2$\alpha$), in which said rating (Rai) decreases as the deviation angle decreases ($\alpha$i) and tends to zero as the deviation angle ($\alpha$i) tends to its optimized value

- wherein assigning a rating comprises:
- (f6) moving the surgical axis (CI, CI_i)
- (f7) calculating a bone height (HR_i; i = 1..n) of said bone;
- (f8) recalculating (CTi, ETi, CWi, EWi) as a function of said variable deviation angle ($\alpha$i; i = 1..n),
- identifying a corresponding optimal implant site (SI);
- identifying corresponding optimal dental implants (BMI).

The optimization module 204 is further configured for:

assigning a rating to said measure of a diameter (DTi) or (EDTi) wherein:

- If said diameter (DTi) or (EDTi) is < than the second predefined diameter threshold value (S_D2) → rating = -1

- If said diameter (DTi) or (EDTi) is < than the third predefined diameter threshold value (S_D3) → rating = -2

and / or
assigning a rating to said height measurement (Lli; ELIi) as a function of a first predefined height threshold value (SL_1)
and / or
assigning a rating (R_RI_RC) to said crown / root ratio (RL_RC), wherein if RL / CL is > = at a first threshold (SRLRC1), (in particular = 1) → the rating is = 0
if (RL / CL) <(SRLRC1) → rating -1;
if RL / CL <(SRLRC2) <(SRLRC1), (in particular SRLRC2 = 0.75) → rating -2;
and / or
assigning a rating (R_PO) to said implant percentage within the bone (PO), wherein:

  if (PO) = 100% → rating = 0;
  if the first threshold (PO1) (95%) <PO <second threshold (PO2) (99%) → rating -1;
  if PO <first threshold (PO1) (95%) → rating -2
  and / or
  assigning a rating (R_DO) to said bone density (PO), wherein:

      if DO> density threshold (SDO) → rating = 0

      if DO <density threshold (SDO) → rating = -1.

In general it should be noted that in the present context as well as in the appended claims, the processing unit 10 is presented as divided into distinct functional modules (memory modules or operating modules) for the sole purpose of describing the functionality in a clear and complete manner.

Actually, this processing unit may be comprised of a single electronic device suitably programmed to perform the functions as described, while the several modules may correspond to hardware entities and/or software routines forming part of the programmed device.

Alternatively or in addition, such functions may be performed by a plurality of electronic devices whereon aforesaid functional modules can be distributed.

The processing unit may further make use of one or more processors for executing the instructions contained in the memory modules.

The above functional modules may further be distributed over several computers locally or remotely on the basis of the network architecture in which they reside.

The systems further comprise all means and/or memory modules and/or operational means needed to implement the functions illustrated in the respective methods as described.A method/system of identifying an optimal dental implant was described which is in accordance with the guidelines and suitable for the anatomy and treatment proposedin respect to optimal available dental implants.

The invention confers the main _technical effect_ of identifying an optimal dental implant in terms of sizing and positioning.

In particular, the invention, as described, achieves the technical effects of:

- identifying an exact dental implant in terms of structure and sizing.
- identifying an objective dental implant in terms of design.
- identifying a durable and effective dental implant.
- identifying a dental implant with ease.

It should be noted that the simulation system/method of the invention can not be performed by purely mental or mathematical means, nor by the thought process that led to the simulation method.

The simulation performed by the invention achieves technical functions typical of modern engineering work. It provides for realistic prediction of the performance of a dental implant in respect to the designed implant site which shall accommodate the former, and thereby ideally allows the dental implant to be developed so accurately such that a prototype's chances of success can be assessed before it is built.

The technical result increases with the speed of the simulation method, as this enables a wide range of designs to be virtually tested and examined for suitability before the expensive implant fabrication process starts. Without a suitable technical support, an advanced test on a complex dental implant and/or a careful selection among many different designs would not be possible, or at least not feasible within a reasonable time.

It follows that computer-implemented simulation methods for virtual testing represent a practical and practice-oriented part of the dentist's or healthcare professional's or treatment provider's toolkit. What makes them so important is the fact that that there isn't a purely mathematical, theoretical or mental method which is capable of providing a complete and / or fast prediction of a dental implant performance according to the parameters of the invention that is different or diversifiable for each single patient.

## Claims

1. Computer-implemented method of identifying, in a patient, at least one dental implant (MI) for an implant site (SI), comprising the steps of:

   (f1) graphically simulating, via a computed tomography (TAC) an anatomy of a envisaged dental prosthesis (PD), a respective edentulous site (LR) corresponding to an identified tooth (Tn) and of a maxillary bone (OM), wherein said envisaged dental prosthesis (PD) is couplable to a dental implant (ID) insertable into the maxillary bone (OM)

   (f2) calculating an ideal prosthetic axis (PI) for said envisaged dental prosthesis (PD) based on said computed tomography (TAC), wherein said ideal prosthetic axis (PI) is an axis crossing an ideal point of the envisaged dental prosthesis (PD) as a function of the type of tooth (Tn) considered;

   (f3) calculating a first cervical distance (CT) and a first apical distance (ET) of said maxillary bone (OM) at said edentulous site (LR) based on said computed tomography (TAC), wherein the distances are calculated in a first direction relative to said maxillary bone (OM), wherein said first cervical distance (CT) is detected at a first cervical portion (q1) with respect to a reference portion.

   (f4) calculating a second cervical distance (CW) and a second apical distance (EW) of said maxillary bone (OM) at said edentulous site (LR) based on said computed tomography (TAC), wherein the distances are calculated in a second direction relative to the maxillary bone OM.

   (f5) calculating an ideal surgical axis (CI) on said maxillary bone (OM) as an axis passing through two points, a first (Cx) obtained as an intersection between representative segments of the cervical distances (CT, CW), and a second (Ex) obtained as an intersection between segments representative of the apical distances (ET,EW), wherein said ideal surgical axis (CI) is an axis configured to perform an ideal halving of the bone quantity of the maxillary bone (OM) at the edentulous site (LR).

   wherein the ideal prosthetic axis (PI) and the ideal surgical axis (CI) determine a reference system (REF_ID) for said dental implant (ID),
   wherein said ideal prosthetic axis (PI) and said ideal surgical axis (CI) are offset by a deviation angle ($\alpha$);

   (f6) calculating a bone height (HR) of said maxillary bone (OM) as a distance on said ideal surgical axis (CI) between said first cervical distance (CT) and said first apical distance (ET), wherein said bone height (HR) is representative of a bone availability of said maxillary bone (OM) at said edentulous site (LR);

(f7) moving said ideal surgical axis (CI) so as to determine a surgical compromise axis (CI_i;i=1..n) corresponding to a variable deviation angle ($\alpha i$; i=1..n) with respect to said ideal prosthetic axis (PI);

(f8) simulating an optimised implant site (SI) in said maxillary bone (OM),

wherein said optimised implant site (SI) has an at least partially cylindrical volumetric shape (VSI) inscribed in a foreseen circumscribing volume (V), said simulation being performed at least as a function of:

o said identified tooth (Tn);
o a said first cervical distance (CTi) and a said first apical distance (ETi);
o a said second cervical distance (CWi) and a said second apical distance (EWi);
o a said first bone height (HRi);
o said variable deviation angle ($\alpha i$);

wherein the optimised implant site (SI) is further simulated as a function of the first representative parameters (PSI) thereof comprising at least:

o a diameter (DTi) of said at least partially cylindrical volumetric shape (VSI);
o a height (Lli) of said at least partially cylindrical volumetric shape (VSI);

wherein said first representative parameters (PSI) have values varying as a function of said variable deviation angle ($\alpha i$);

(f9) providing a database (BD_IE) comprising second representative parameters (PIE) of existing dental implants (IE);
(f10) comparing said first representative parameters (PSI) with said second representative parameters (PIE);
(f11) identifying one or more dental implants (Mli) for said optimised implant site (SI) as a function of the comparison between said first representative parameters (PSI) and said second representative parameters (PIE),

wherein:

- in said step (f8) of simulating said implant site (SI), said first representative parameters (PSI) further comprise:
o one or more rating values (Rai,RDT,RLI,RRLRC,RPO,RDO) of said optimised implant site (SI) defined as a function of a comparison between said first representative parameters (PSI) and reference threshold values;

wherein said optimised implant site (SI) is identified with a rating (R) defined as a combined function of said rating values (Rai, RDT, RLI, RRLRC, RPO, RDO), wherein said step (f11) identifies one or more optimal dental implants (BMli) as a function of the comparison between said first representative parameters (PSI) and said second representative parameters (PIE) when said rating (R) is minimised, wherein said first representative parameters (PSI) and said second representative parameters (PIE) comprise respectively said diameter (DTi) of said at least partially cylindrical volumetric shape (VSI) and said implant diameter (EDTi) for the existing implant (IE).

2. Computer-implemented method according to claim 1 wherein:

said first representative parameters (PSI) and said second representative parameters (PIE) comprise respectively said diameter (DTi) of said at least partially cylindrical volumetric shape (VSI) and said implant diameter (EDTi) for the existing implant (IE) obtained with the sub-steps of:

- calculating a minimum measurement (MIN_L) between CT, MT, ET and CW, MW, EW
- calculating said diameter (DTi; EDTi) as a function of the calculated minimum measurement (MIN_L) and of a first predefined diameter threshold value (S_D1);
- checking that (DTi) or (EDTi) is at distance > = than a first thickness threshold (S_VE) with respect to the vestibular peak (PV);
- checking that (DTi) or (EDTi) is at distance > = than a second thickness threshold (S_PL) with respect to the lingual peak (PL) and / or

wherein said first representative parameters (PSI) and said second representative parameters (PIE) comprise respectively said height (Lli) of said at least partially cylindrical volumetric form () and said implant height (ELli) for the existing plant (IE), obtained as a function of a bone (HR_i) height value
and/or
wherein said first representative parameters (PSI) and said second representative parameters (PIE) comprise a crown / root ratio and / or wherein said first representative parameters (PSI) and said second representative parameters (PIE) comprise a percentage (PO) of implant within the maxillary bone (OM) and / or
wherein said first representative parameters (PSI) and said second representative parameters (PIE) comprise a bone density (DO).

3. Computer-implemented method according to any one of the preceding claims, wherein the database (BD_IE) comprises said second representative parameters (PID) representative of:

   ○ a tooth identifier (ETn) for an existing implant (IE);
   ○ an implant diameter (EDTi) for an existing implant (IE);
   ○ an implant height (ELIi) for an existing implant (IE);
   ○ a deviation angle (Eai) for an existing implant (IE);
   ○ a rating identifier (ERi) for an existing implant (IE);
   ○ implant manufacturer;
   ○ implant model.

4. Computer-implemented method according to any one of the preceding claims, wherein said comparison between said first representative parameters (PSI) and said second representative parameters (PIE) is performed as a function of a different comparison priority (Pconf).

5. Computer-implemented method according to any one of claims 1 to 4, wherein said step of minimising said rating (R) comprises:
   ○ (f12) comparing said deviation angle ($\alpha i$; i=1..n) with predefined threshold values of deviation angles (S1$\alpha$; S2$\alpha$) comprising a first predefined deviation angle threshold value (S1$\alpha$) and a second predefined deviation angle threshold value (S2$\alpha$), where (S1$\alpha$) < (S2$\alpha$).

6. Computer-implemented method according to claim 5, wherein said step (f12) of comparing said deviation angle ($\alpha i$; i=1..n) comprises the steps of:

   (f6) recalculating said bone thickness (HR_i);
   (f7) moving said surgical axis (CI, CI_i) with respect to said ideal prosthetic axis (PI_i) maintained in a fixed position, so as to bring about a reduction in said variable deviation angle ($\alpha i$;

i=1..n);
wherein the steps described are carried out as long as the variable deviation angle ($\alpha i$) remains greater than said first default threshold value of the deviation angle (S1$\alpha$), said deviation angle ($\alpha i$; i=1..n) being minimised when it is less than said first default threshold value of the deviation angle (S1$\alpha$).

7. Computer-implemented method according to claim 6 comprising the steps of:

   - (f13) assigning a rating (Rai) to said implant site (SI) as a function of said step (f12) of comparing said variable deviation angle ($\alpha i$; i=1..n) with the default threshold value of the deviation angle (S1$\alpha$) wherein said rating (Rai) decreases as the deviation angle ($\alpha i$) decreases and tends towards zero as the deviation angle ($\alpha i$) tends towards its optimised value wherein said step (f13) of assigning a rating includes the steps of:
   - (f6) moving the surgical axis (CI, CI_i)
   - (f7) calculating a bone height (HR_i; i = 1..n) of said bone;
   - (f8) recalculating (CTi, ETi, CWi, EWi) as a function of said variable deviation angle ($\alpha i$; i = 1..n),
   - identifying a corresponding optimal implant site (SI);
   - identifying corresponding optimal dental implants (BMI).

8. Computer-implemented method according to any one of claims 3 to 7 comprising the step of assigning a rating to said measure of a diameter (DTi) or (EDTi) wherein:

   - If said diameter (DTi) or (EDTi) is < than the second default diameter threshold value (S_D2) → rating = -1
   - If said diameter (DTi) or (EDTi) is < than the third default diameter threshold value (S_D3) → rating = -2 and / or

     assigning a rating to said height measurement (LIi; ELIi) as a function of a first default height threshold value (SL_1) and / or
     assigning a rating (R_RI_RC) to said crown / root ratio (RL_RC), in which if RL / CL> = at a first threshold (SRLRC1), (in particular = 1) → the rating = 0
     if (RL / CL) < (SRLRC1 → rating -1;
     if RL / CL < (SRLRC2) < (SRLRC1), (in particular SRLRC2 = 0.75) → rating -2;
     and / or
     assigning a rating (R_PO) to said implant percentage within the bone (PO), wherein:

if (PO) = 100% → rating = 0;
if the first threshold (PO1) (95%) <PO <second threshold (PO2) (99%) → rating -1;
if PO <first threshold (PO1) (95%) → rating -2
and / or
assigning a rating (R_DO) to said bone density (PO), wherein:

> if DO> density threshold (SDO) → rating = 0
> if DO <density threshold (SDO) → rating = -1.

9. Computer program configured for, when in use, performing the computer-implemented method of one of the preceding claims.

10. Identification system of at least one dental implant (MI) for an implant site (SI) including:

> Computer-implemented simulation graphic means (1) configured to graphically simulate via a computed tomography (TAC) an anatomy of a provided dental prosthesis (PD), a respective edentulous site (LR) corresponding to an identified tooth (Tn) and of a maxillary bone (OM), wherein said provided dental prosthesis (PD) is coupleable to a dental implant (ID) which is inseartable into the maxillary bone (OM);
> a processing unit (10) configured to simulate an optimized implant site (SI) starting from the simulation of the anatomy of the provided dental prosthesis (PD), in which the processing unit 10 comprises:
>
>> - a first calculation module (101) configured to calculate an ideal prosthetic axis (PI) for the provided dental prosthesis (PD) based on said computed tomography (TAC) wherein said ideal prosthetic axis (PI) is an axis crossing an ideal point of the provided dental prosthesis (PD) as a function of the type of tooth (Tn) considered;
>> - a second calculation module (102) configured to calculate a first cervical distance (CT) and a first apical distance (ET) of said maxillary bone (OM) at said edentulous site (LR) based on said computed tomography (TAC), in which the distances are calculated in a first direction with respect to said maxillary bone (OM), wherein said first cervical distance (CT) is detected at a first cervical portion (q1) relative to a reference portion;
>> - a third calculation module (103) configured to calculate a second cervical distance (CW) and a second apical distance (EW) of said maxillary bone (OM) at said edentulous site (LR) based on said computed tomography (TAC), in which the distances are calculated in a second direction with respect to the maxillary bone OM;
- a fourth calculation module (104) configured to calculate an ideal surgical axis (CI) on said maxillary bone (OM) as an axis passing through two points, a first (Cx) obtained as an intersection between representative segments of the cervical distances (CT, CW), and a second (Ex) obtained as an intersection between segments representative of the apical distances (ET,EW), wherein said ideal surgical axis (CI) is an axis configured to perform an ideal halving of the bone quantity of the maxillary bone (OM) at the edentulous site (LR);

wherein the ideal prosthetic axis (PI) and the ideal surgical axis (CI) determine a reference system (REF_ID) for said dental implant (ID); wherein said ideal prosthetic axis (PI) and said ideal surgical axis (CI) are offset by a deviation angle ($\alpha$);

> - a fifth calculation module (105) configured to calculate a bone height (HR) of said maxillary bone (OM) as a distance on said ideal surgical axis (CI) between said first cervical distance (CT) and said first apical distance (ET), in which said bone height (HR) is representative of a bone availability of said maxillary bone (OM) at said edentulous site (LR);
> - a movement module (106) configured to move said ideal surgical axis (CI) by determining a compromising surgical axis (CI_i; i = 1..n) corresponding to a variable deviation angle ($\alpha i$; i = 1..n ) with respect to said ideal prosthetic axis (PI);
> - a simulation module (107B) configured to simulate said optimized implant site (SI) in said maxillary bone (OM), in which said optimized implant site (SI) has at least partially cylindrical volumetric shape (VSI) inscribed within an expected circumscribing volume (V), said simulation being performed at least as a function of:
>
>> • said identified tooth (Tn);
>> • a said first cervical distance (CTi) and a said first apical distance (ETi);
>> • a said second cervical distance (CWi) and a said second apical distance (EWi);
>> • a said first bone height (HRi);
>> • said variable deviation angle ($\alpha i$);

- wherein the optimized implant site (SI) is at least simulated as a function of its first representative parameters (PSI) including at least:

    • a diameter (DTi) of said at least partially cylindrical volumetric shape (VSI)
    • a height (Lli) of said at least partially cylindrical volumetric shape (VSI);

wherein said first representative parameters (PSI) have variable values as a function of said variable deviation angle ($\alpha i$);

- a database (BD_IE) including second representative parameters (PIE) of existing dental implants (IE), a second processing unit (20) comprising:
- a comparison module (201) configured to compare said first representative parameters (PSI) with said second representative parameters (PIE);
- a first identification module (202) configured to identify one or more dental implants (Mli) for said optimized implant site (SI) as a function of the comparison performed by the comparison module (201);

wherein said first representative parameters (PSI) further comprise one or more rating values (Rai, RDT, RLI, RRLRC, RPO, RDO) of said optimized implant site (SI) defined as a function of a comparison between the first representative PSI parameters and reference threshold values; wherein the optimized implant site SI is identified with a rating R defined as a combined function of said rating values (Rai, RDT, RLI, RRLRC, RPO, RDO),

- a second identification module (203) configured to identify one or more optimal dental implants (BMli) as a function of the comparison performed by said comparison module (201) when said rating (R) is minimized, wherein said first representative parameters (PSI) and said second representative parameters (PIE) comprise respectively said diameter (DTi) of said at least partially cylindrical volumetric shape (VSI) and said implant diameter (EDTi) for each existing plant (IE).

11. Identification system according to claim 10 wherein said first representative parameters (PSI) and said second representative parameters (PIE) comprise respectively said diameter (DTi) of said at least partially cylindrical volumetric shape (VSI) and said implant diameter (EDTi) for each existing plant (IE) obtained with the sub-steps of:

- calculating a minimum measurement (MIN_L) between CT, MT, ET and CW, MW, EW
- calculating said diameter (DTi; EDTi) as a function of the calculated minimum measurement (MIN_L) and of a first default diameter threshold value (S_D1);
- checking that (DTi) or (EDTi) is at a distance> = of a first thickness threshold (S_VE) with respect to the vestibular peak (PV);
- checking that (DTi) or (EDTi) is at distance> = of a second thickness threshold (S_PL) with respect to the lingual peak (PL) and / or
wherein said first representative parameters (PSI) and said second representative parameters (PIE) comprise respectively the height (Lli) of said at least partially cylindrical volumetric form () and said implant height (ELli) for each existing plant (IE), obtained as a function of a bone height value (HR_i)
and / or
wherein said first representative parameters (PSI) and said second representative parameters (PIE) comprise a crown / root ratio and / or
wherein said first representative parameters (PSI) and said second representative parameters (PIE) comprise a percentage (PO) of the implant within the maxillary bone (OM)
and / or
wherein said first representative parameters (PSI) and said second representative parameters (PIE) comprise a bone density (DO).

12. Identification system according to any one of claims 10 to 11, wherein the data base (BD_IE) comprises said second representative parameters (PID) representative of:

    • a tooth identifier (ETn) for each existing implant (IE);
    • a implant diameter (EDTi) for each existing implant (IE);
    • a implant height (ELli) for each existing implant (IE);
    • a deviation angle (Eai) for each existing implant (IE);
    • a rating identifier (ERi) for each existing implant (IE);
    • implant house;
    • implant model.

13. Identification system according to any one of claims 10 to 12, wherein said comparison module (201) is configured to compare said first representative parameters (PSI) and said second representative parameters (PIE) as a function of a different comparison priority (Pconf).

**14.** Identification system according to any one of claims 10 to 13, wherein said step of minimizing said rating (R) comprises:

    • (f12) comparing said deviation angle ($\alpha$i; i = 1..n) with default threshold values of deviation angles (S1$\alpha$; S2$\alpha$) including a first default deviation angle (S1$\alpha$) threshold value and a second default threshold value of deviation angles (S2$\alpha$), where (S1$\alpha$) <(S2$\alpha$).

**Patentansprüche**

**1.** Computerimplementiertes Verfahren zur Identifizierung mindestens eines Zahnimplantats (MI) für eine Implantationsstelle (SI) bei einem Patienten, umfassend die Schritte:

    (f1) grafisches Simulieren über eine Computertomographie (TAC) einer Anatomie einer vorgesehenen Zahnprothese (PD), einer jeweiligen zahnlosen Stelle (LR), die einem identifizierten Zahn (Tn) entspricht und eines Oberkieferknochens (OM), wobei die vorgesehene Zahnprothese (PD) an ein in den Oberkieferknochen (OM) einsetzbares Zahnimplantat (ID) koppelbar ist;

    (f2) Berechnen einer idealen Prothesenachse (PI) für die vorgesehene Zahnprothese (PD) basierend auf der Computertomographie (TAC), wobei es sich bei der idealen Prothesenachse (PI) um eine Achse handelt, die einen idealen Punkt der vorgesehenen Zahnprothese (PD) als Funktion des betrachteten Zahntyps (Tn) schneidet;

    (f3) Berechnen einer ersten zervikalen Distanz (CT) und einer ersten apikalen Distanz (ET) des Oberkieferknochens (OM) an der zahnlosen Stelle (LR) basierend auf der Computertomographie (TAC), wobei die Distanzen in einer ersten Richtung relativ zu dem Oberkieferknochen (OM) berechnet werden, wobei die erste zervikale Distanz (CT) an einem ersten zervikalen Abschnitt (q1) in Bezug auf einen Referenzabschnitt erfasst wird;

    (f4) Berechnen einer zweiten zervikalen Distanz (CW) und einer zweiten apikalen Distanz (EW) des Oberkieferknochens (OM) an der zahnlosen Stelle (LR) basierend auf der Computertomographie (TAC), wobei die Distanzen in einer zweiten Richtung relativ zum Oberkieferknochen OM berechnet werden;

    (f5) Berechnen einer idealen chirurgischen Achse (CI) auf dem Oberkieferknochen (OM) als eine Achse, die durch zwei Punkte verläuft, einen ersten (Cx), der als Schnittpunkt zwischen repräsentativen Segmenten der zervikalen Distanzen (CT, CW) erhalten wird, und einen zweiten (Ex), der als Schnittpunkt zwischen Segmenten, die für die apikalen Distanzen (ET, EW) repräsentativ sind, erhalten wird, wobei es sich bei der idealen chirurgischen Achse (CI) um eine Achse handelt, die konfiguriert ist, um eine ideale Halbierung der Knochenmenge des Oberkieferknochens (OM) an der zahnlosen Stelle (LR) durchzuführen,

    wobei die ideale Prothesenachse (PI) und die ideale chirurgische Achse (CI) ein Referenzsystem (REF_ID) für das Zahnimplantat (ID) bestimmen,
    wobei die ideale Prothesenachse (PI) und die ideale chirurgische Achse (CI) um einen Abweichungswinkel (a) versetzt sind;

    (f6) Berechnen einer Knochenhöhe (HR) des Oberkieferknochens (OM) als Distanz auf der idealen chirurgischen Achse (CI) zwischen der ersten zervikalen Distanz (CT) und der ersten apikalen Distanz (ET), wobei die Knochenhöhe (HR) repräsentativ für eine Knochenverfügbarkeit des Oberkieferknochens (OM) an der zahnlosen Stelle (LR) ist;
    (f7) Bewegen der idealen chirurgischen Achse (CI), um eine chirurgische Kompromissachse (CI_i; i=1 .. n) entsprechend einem variablen Abweichungswinkel ($\alpha$i; i=1 .. n) in Bezug auf die ideale Prothesenachse (PI) zu bestimmen;
    (f8) Simulieren einer optimierten Implantationsstelle (SI) in dem Oberkieferknochen (OM), wobei die optimierte Implantationsstelle (SI) eine zumindest teilweise zylindrische volumetrische Form (VSI) aufweist, die in ein vorgesehenes umschreibendes Volumen (V) eingeschrieben ist,

    wobei die Simulation ausgeführt ist, mindestens als Funktion von:

        o dem identifizierten Zahn (Tn);
        o der ersten zervikalen Distanz (CTi) und der ersten apikalen Distanz (ETi);
        o der zweiten zervikalen Distanz (CWi) und der zweiten apikalen Distanz (EWi);
        o der ersten Knochenhöhe (HRi);
        o dem variablen Abweichungswinkel ($\alpha$i);

    wobei die optimierte Implantationsstelle (SI) ferner als Funktion der ersten repräsentativen Parameter (PSI) davon simuliert wird, umfassend mindestens:

        o einen Durchmesser (DTi) der zumindest teilweise zylindrischen volumetrischen Form (VSI);

o eine Höhe (LIi) der zumindest teilweise zylindrischen volumetrischen Form (VSI);

wobei die ersten repräsentativen Parameter (PSI) Werte aufweisen, die als Funktion des variablen Abweichungswinkels ($\alpha i$) variieren;

(f9) Bereitstellen einer Datenbank (BD_IE), die zweite repräsentative Parameter (PIE) bestehender Zahnimplantate (IE) umfasst;

(f10) Vergleichen der ersten repräsentativen Parameter (PSI) mit den zweiten repräsentativen Parametern (PIE);

(f11) Identifizieren eines oder mehrerer Zahnimplantate (MIi) für die optimierte Implantationsstelle (SI) als Funktion des Vergleichs zwischen den ersten repräsentativen Parametern (PSI) und den zweiten repräsentativen Parametern (PIE),

wobei:

- im Schritt (f8) zum Simulieren der Implantationsstelle (SI) die ersten repräsentativen Parameter (PSI) ferner umfassen:

o einen oder mehrere Bewertungswerte (Rai,RDT,RLI,RRLRC,RPO,RDO) der optimierten Implantationsstelle (SI), die als Funktion eines Vergleichs zwischen den ersten repräsentativen Parametern (PSI) und Referenzschwellenwerten definiert sind;

wobei die optimierte Implantationsstelle (SI) mit einer Bewertung (R) identifiziert wird, die als eine kombinierte Funktion der Bewertungswerte (Rai, RDT, RLI, RRLRC, RPO, RDO) definiert ist, wobei der Schritt (f11) ein oder mehrere optimale Zahnimplantate (BMIi) als Funktion des Vergleichs zwischen den ersten repräsentativen Parametern (PSI) und den zweiten repräsentativen Parametern (PIE) identifiziert, wenn die Bewertung (R) minimiert wird, wobei die ersten repräsentativen Parameter (PSI) und die zweiten repräsentativen Parameter (PIE) jeweils den Durchmesser (DTi) der zumindest teilweise zylindrischen volumetrischen Form (VSI) und den Implantatdurchmesser (EDTi) für das bestehende Implantat (IE) umfassen.

**2.** Computerimplementiertes Verfahren nach Anspruch 1, wobei:

die ersten repräsentativen Parameter (PSI) und die zweiten repräsentativen Parameter (PIE) jeweils den Durchmesser (DTi) der zumindest teilweise zylindrischen volumetrischen Form (VSI) und den Implantatdurchmesser (EDTi) für das bestehende Implantat (IE) umfassen, die mit den Teilschritten erhalten wurden:

- Berechnen einer minimalen Messung (MIN_L) zwischen CT, MT, ET und CW, MW, EW;
- Berechnen des Durchmessers (DTi; EDTi) als Funktion der berechneten minimalen Messung (MIN_L) und eines ersten vordefinierten Durchmesserschwellenwerts (S_D1);
- Prüfen, ob sich (DTi) oder (EDTi) bei einer Distanz > = als ein erster Dickenschwellenwert (S_VE) in Bezug auf die vestibuläre Spitze (PV) befindet;
- Prüfen, ob sich (DTi) oder (EDTi) bei einer Distanz > = als ein zweiter Dickenschwellenwert (S_PL) in Bezug auf die linguale Spitze (PL) befindet

und/oder
wobei die ersten repräsentativen Parameter (PSI) und die zweiten repräsentativen Parameter (PIE) jeweils die Höhe (LIi) der zumindest teilweise zylindrischen volumetrischen Form und die Implantathöhe (ELIi) für das bestehende Implantat (IE) umfassen, die als Funktion eines Knochenhöhenwertes (HR_i) erhalten wurden und/oder
wobei die ersten repräsentativen Parameter (PSI) und die zweiten repräsentativen Parameter (PIE) ein Kronen/Wurzel-Verhältnis umfassen
und/oder
wobei die ersten repräsentativen Parameter (PSI) und die zweiten repräsentativen Parameter (PIE) einen Prozentsatz (PO) des Implantats innerhalb des Oberkieferknochens (OM) umfassen
und/oder
wobei die ersten repräsentativen Parameter (PSI) und die zweiten repräsentativen Parameter (PIE) eine Knochendichte (DO) umfassen.

**3.** Computerimplementiertes Verfahren nach einem der vorhergehenden Ansprüche,
wobei die Datenbank (BD_IE) die zweiten repräsentativen Parameter (PID) umfasst, die repräsentativ sind für:

o einen Zahnidentifikator (ETn) für ein bestehendes Implantat (IE);
o einen Implantatdurchmesser (EDTi) für ein bestehendes Implantat (IE);
o eine Implantathöhe (ELIi) für ein bestehendes Implantat (IE);
o einen Abweichungswinkel (Eai) für ein bestehendes Implantat (IE);
o einen Bewertungsidentifikator (ERi) für ein bestehendes Implantat (IE);
o Hersteller von Implantaten;
o Implantatmodell.

4. Computerimplementiertes Verfahren nach einem der vorhergehenden Ansprüche, wobei der Vergleich zwischen den ersten repräsentativen Parametern (PSI) und den zweiten repräsentativen Parametern (PIE) als Funktion einer unterschiedlichen Vergleichspriorität (Pconf) ausgeführt wird.

5. Computerimplementiertes Verfahren nach einem der Ansprüche 1 bis 4, wobei der Schritt zum Minimieren der Bewertung (R) umfasst:

o (f12) Vergleichen des Abweichungswinkels ($\alpha$i; i=1 .. n) mit vordefinierten Schwellenwerten von Abweichungswinkeln (S1$\alpha$; S2$\alpha$), umfassend einen ersten vordefinierten Abweichungswinkelschwellenwert (S1$\alpha$) und einen zweiten vordefinierten Abweichungswinkelschwellenwert (S2a), wo (S1$\alpha$) < (S2a).

6. Computerimplementiertes Verfahren nach Anspruch 5, wobei der Schritt (f12) zum Vergleichen des Abweichungswinkels ($\alpha$i; i=1 .. n) die Schritte umfasst:

(f6) Neuberechnen der Knochendicke (HR_i);
(f7) Bewegen der chirurgischen Achse (CI, CI_i) in Bezug auf die in einer festen Position gehaltene ideale Prothesenachse (PI_i), um eine Verringerung des variablen Abweichungswinkels ($\alpha$i; i=1 .. n) zu bewirken; wobei die beschriebenen Schritte ausgeführt werden, solange der variable Abweichungswinkel ($\alpha$i) größer als der erste Default-Schwellenwert des Abweichungswinkels (S1$\alpha$) bleibt, wobei der Abweichungswinkel ($\alpha$i; i=1 .. n) minimiert wird, wenn er kleiner ist als der erste Default-Schwellenwert des Abweichungswinkels (S1$\alpha$).

7. Computerimplementiertes Verfahren nach Anspruch 6, umfassend die Schritte:

- (f13) Zuweisen einer Bewertung (Rai) zu der Implantationsstelle (SI) als Funktion des Schrittes (f12) zum Vergleichen des variablen Abweichungswinkels ($\alpha$i; i=1 .. n) mit dem Default-Schwellenwert des Abweichungswinkels (S1$\alpha$),

wobei die Bewertung (Rai) abnimmt, wenn der Abweichungswinkel ($\alpha$i) abnimmt und gegen Null tendiert, wenn der Abweichungswinkel ($\alpha$i) zu seinem optimierten Wert tendiert, wobei der Schritt (f13) zum Zuweisen einer Bewertung die Schritte umfasst:
- (f6) Bewegen der chirurgischen Achse (CI, CI_i)
- (f7) Berechnen einer Knochenhöhe (HR_i; i = 1 .. n) des Knochens;
- (f8) Neuberechnen (CTi, ETi, CWi, EWi) als Funktion des variablen Abweichungswinkels ($\alpha$i; i=1 .. n),
- Identifizieren einer entsprechenden optimalen Implantationsstelle (SI);
- Identifizieren entsprechender optimaler Zahnimplantate (BMI).

8. Computerimplementiertes Verfahren nach einem der Ansprüche 3 bis 7, umfassend den Schritt zum Zuweisen einer Bewertung zu dem Maß eines Durchmessers (DTi) oder (EDTi), wobei:

- Wenn der Durchmesser (DTi) oder (EDTi) < als der zweite Default-Durchmesserschwellenwert (S_D2) ist → Bewertung = -1
- Wenn der Durchmesser (DTi) oder (EDTi) < als der dritte Default-Durchmesserschwellenwert (S_D3) ist → Bewertung = -2
und/oder

Zuweisen einer Bewertung zu der Höhenmessung (LIi; ELIi) als Funktion eines ersten Default-Höhenschwellenwerts (SL_1)
und/oder
Zuweisen einer Bewertung (R_RI_RC) zu dem Kronen / Wurzel-Verhältnis (RL_RC), wobei
wenn RL / CL> = bei einem ersten Schwellenwert (SRLRC1), (insbesondere = 1) → Bewertung =0
wenn (RL / CL) <(SRLRC1) → Bewertung -1;
wenn RL / CL <(SRLRC2) <(SRLRC1), (insbesondere SRLRC2 = 0,75) → Bewertung -2;
und/oder
Zuweisen einer Bewertung (R_PO) zu dem Implantatprozentsatz innerhalb des Knochens (PO), wobei:

wenn (PO) = 100% → Bewertung = 0;
wenn der erste Schwellenwert (PO1) (95%) <PO <zweiter Schwellenwert (PO2) (99%) → Bewertung -1;
wenn PO <erster Schwellenwert (PO1) (95%) → Bewertung - 2
und/oder

Zuweisen einer Bewertung (R_DO) zu der Knochendichte (PO), wobei:

wenn DO> Dichteschwellenwert (SDO) → Bewertung = 0
wenn DO <Dichteschwellenwert (SDO) → Bewertung = -1.

9. Computerprogramm, das konfiguriert ist, bei Verwendung das computerimplementierte Verfahren nach einem der vorhergehenden Ansprüche durchzuführen.

10. Identifizierungssystem von mindestens einem Zahnimplantat (MI) für eine Implantationsstelle (SI) enthaltend:

computerimplementierte grafische Simulationsmittel (1), die konfiguriert sind, um über eine Computertomographie (TAC) eine Anatomie einer bereitgestellten Zahnprothese (PD), einer jeweiligen zahnlosen Stelle (LR), die einem identifizierten Zahn (Tn) entspricht, und eines Oberkieferknochens (OM) grafisch zu simulieren, wobei die bereitgestellte Zahnprothese (PD) mit einem in den Oberkieferknochen (OM) einsetzbaren Zahnimplantat (ID) koppelbar ist; eine Verarbeitungseinheit (10), die konfiguriert ist, um ausgehend von der Simulation der Anatomie des bereitgestellten Zahnimplantats (PD) eine optimierte Implantationsstelle (SI) zu simulieren, wobei die Verarbeitungseinheit 10 umfasst:

- ein erstes Berechnungsmodul (101), das konfiguriert ist, um eine ideale Prothesenachse (PI) für die bereitgestellte Zahnprothese (PD) basierend auf der Computertomographie (TAC) zu berechnen, wobei es sich bei der idealen Prothesenachse (PI) um eine Achse handelt, die einen idealen Punkt der bereitgestellten Zahnprothese (PD) als Funktion des betrachteten Zahntyps (Tn) schneidet;
- ein zweites Berechnungsmodul (102), das konfiguriert ist, um eine erste zervikale Distanz (CT) und eine erste apikale Distanz (ET) des Oberkieferknochens (OM) an der zahnlosen Stelle (LR) basierend auf der Computertomographie (TAC) zu berechnen, wobei die Distanzen in einer ersten Richtung in Bezug auf den Oberkieferknochen (OM) berechnet werden, wobei die erste zervikale Distanz (CT) an einem ersten zervikalen Abschnitt (q1) relativ zu einem Referenzabschnitt erfasst wird;
- ein drittes Berechnungsmodul (103), das konfiguriert ist, um eine zweite zervikale Distanz (CW) und eine zweite apikale Distanz (EW) des Oberkieferknochens (OM) an der zahnlosen Stelle (LR) basierend auf der Computertomographie (TAC) zu berechnen, wobei die Distanzen in einer zweiten Richtung in Bezug auf den Oberkieferknochen OM berechnet werden;
- ein viertes Berechnungsmodul (104), das konfiguriert ist, um eine ideale chirurgische Achse (CI) auf dem Oberkieferknochen (OM) als eine Achse zu berechnen, die durch zwei Punkte verläuft, einen ersten (Cx), der als Schnittpunkt zwischen repräsentativen Segmenten der zervikalen Distanzen (CT, CW) erhalten wird, und einen zweiten (Ex), der als Schnittpunkt zwischen Segmenten, die für die apikalen Distanzen (ET, EW) repräsentativ sind, erhalten wird, wobei es sich bei der idealen chirurgischen Achse (CI) um eine Achse handelt, die konfiguriert ist, um eine ideale Halbierung der Knochenmenge des Oberkieferknochens (OM) an der zahnlosen Stelle (LR) durchzuführen;

wobei die ideale Prothesenachse (PI) und die ideale chirurgische Achse (CI) ein Referenzsystem (REF_ID) für das Zahnimplantat (ID) bestimmen;
wobei die ideale Prothesenachse (PI) und die ideale chirurgische Achse (CI) um einen Abweichungswinkel (a) versetzt sind;

- ein fünftes Berechnungsmodul (105), das konfiguriert ist, um eine Knochenhöhe (HR) des Oberkieferknochens (OM) als eine Distanz auf der idealen chirurgischen Achse (CI) zwischen der ersten zervikalen Distanz (CT) und der ersten apikalen Distanz (ET) zu berechnen, wobei die Knochenhöhe (HR) repräsentativ für eine Knochenverfügbarkeit des Oberkieferknochens (OM) an der zahnlosen Stelle (LR) ist;
- ein Bewegungsmodul (106), das konfiguriert ist, um die ideale chirurgische Achse (CI) durch Bestimmen einer kompromittierenden chirurgischen Achse (CI_i; i = 1 .. n) entsprechend einem variablen Abweichungswinkel ($\alpha$i; i=1 .. n ) mit Bezug auf die ideale Prothesenachse (PI) zu bewegen;
- ein Simulationsmodul (107B), das konfiguriert ist, um die optimierte Implantationsstelle (SI) in dem Oberkieferknochen (OM) zu simulieren, wobei die optimierte Implantationsstelle (SI) eine zumindest teilweise zylindrische volumetrische Form (VSI) aufweist, die innerhalb eines geplanten umschreibenden Volumens (V) eingeschrieben ist, wobei die Simulation mindestens ausgeführt ist, als Funktion von:

- dem identifizierten Zahn (Tn);
- der ersten zervikalen Distanz (CTi) und der ersten apikalen Distanz (ETi);
- der zweiten zervikalen Distanz (CWi) und der zweiten apikalen Distanz (EWi);
- der ersten Knochenhöhe (HRi);
- dem variablen Abweichungswinkel (ai);

- wobei die optimierte Implantationsstelle (SI) zumindest als Funktion ihrer ersten repräsentativen Parameter (PSI) simuliert wird, enthaltend zumindest:

- einen Durchmesser (DTi) der zumindest teilweise zylindrischen volumetrischen Form (VSI);
- eine Höhe (LIi) der zumindest teilweise zylindrischen volumetrischen Form (VSI);

wobei die ersten repräsentativen Parameter (PSI) variable Werte als Funktion des variablen Abweichungswinkels ($\alpha$i) aufweisen;

- eine Datenbank (BD_IE), die zweite repräsentative Parameter (PIE) bestehender Zahnimplantate (IE) enthält, wobei eine zweite Verarbeitungseinheit (20) umfasst:
- ein Vergleichsmodul (201), das konfiguriert ist, um die ersten repräsentativen Parameter (PSI) mit den zweiten repräsentativen Parametern (PIE) zu vergleichen;
- ein erstes Identifizierungsmodul (202), das konfiguriert ist, um ein oder mehrere Zahnimplantate (MIi) für die optimierte Implantationsstelle (SI) als Funktion des Vergleichs, der von dem Vergleichsmodul (201) ausgeführt wird, zu identifizieren;

wobei die ersten repräsentativen Parameter (PSI) ferner einen oder mehrere Bewertungswerte (Rai, RDT, RLI, RRLRC, RPO, RDO) der optimierten Implantationsstelle (SI) umfassen, definiert als Funktion eines Vergleichs zwischen den ersten repräsentativen PSI-Parametern und Referenzschwellenwerten;
wobei die optimierte Implantationsstelle SI mit einer Bewertung R identifiziert wird, die als eine kombinierte Funktion der Bewertungswerte (Rai, RDT, RLI, RRLRC, RPO, RDO) definiert ist,

- ein zweites Identifizierungsmodul (203), das konfiguriert ist, um ein oder mehrere optimale Zahnimplantate (BMIi) als Funktion des Vergleichs zu identifizieren, der von dem Vergleichsmodul (201) ausgeführt wird, wenn die Bewertung (R) minimiert wird, wobei die ersten repräsentativen Parameter (PSI) und die zweiten repräsentativen Parameter (PIE) jeweils den Durchmesser (DTi) der zumindest teilweise zylindrischen volumetrischen Form (VSI) und den Implantatdurchmesser (EDTi) für ein jedes bestehende Implantat (IE) umfassen.

11. Identifizierungssystem nach Anspruch 10, wobei die ersten repräsentativen Parameter (PSI) und die zweiten repräsentativen Parameter (PIE) jeweils den Durchmesser (DTi) der zumindest teilweise zylindrischen volumetrischen Form (VSI) und den Implantatdurchmesser (EDTi) für ein jedes bestehende Implantat (IE) umfassen, die mit den Teilschritten erhalten wurde:

- Berechnen einer minimalen Messung (MIN_L) zwischen CT, MT, ET und CW, MW, EW
- Berechnen des Durchmessers (DTi; EDTi) als Funktion der berechneten minimalen Messung (MIN_L) und eines ersten Default-Durchmesserschwellenwerts (S_D1);
- Prüfen, ob sich (DTi) oder (EDTi) bei einer Distanz > = als ein erster Dickenschwellenwert (S_VE) in Bezug auf die vestibulären Spitze (PV) befindet;
- Prüfen, ob sich (DTi) oder (EDTi) bei einer Distanz > = als ein zweiter Dickenschwellenwert (S_PL) in Bezug auf die linguale Spitze (PL) befindet
und/oder

wobei die ersten repräsentativen Parameter (PSI) und die zweiten repräsentativen Parameter (PIE) jeweils die Höhe (LIi) der zumindest teilweise zylindrischen volumetrischen Form und die Implantathöhe (ELIi) für ein jedes bestehende Implantat (IE) umfassen, die als Funktion eines Knochenhöhenwertes (HR_i) erhalten wurden
und/oder
wobei die ersten repräsentativen Parameter (PSI) und die zweiten repräsentativen Parameter (PIE) ein Kronen/Wurzel-Verhältnis umfassen
und/oder
wobei die ersten repräsentativen Parameter (PSI) und die zweiten repräsentativen Parameter (PIE) einen Prozentsatz (PO) des Implantats innerhalb des Oberkieferknochens (OM) umfassen

und/oder
wobei die ersten repräsentativen Parameter (PSI) und die zweiten repräsentativen Parameter (PIE) eine Knochendichte (DO) umfassen.

12. Identifizierungssystem nach einem der Ansprüche 10 bis 11, wobei die Datenbank (BD_IE) die zweiten repräsentativen Parameter (PID) umfasst, die repräsentativ sind für:

   • einen Zahnidentifikator (ETn) für ein jedes bestehende Implantat (IE);
   • ·einen Implantatdurchmesser (EDTi) für ein jedes bestehende Implantat (IE);
   • ·eine Implantathöhe (ELIi) für ein jedes bestehende Implantat (IE);
   • einen Abweichungswinkel (Eai) für ein jedes bestehende Implantat (IE);
   • einen Bewertungsidentifikator (ERi) für ein jedes bestehende Implantat (IE);
   • Implantathaus;
   • Implantatmodell.

13. Identifizierungssystem nach einem der Ansprüche 10 bis 12, wobei das Vergleichsmodul (201) konfiguriert ist, um die ersten repräsentativen Parameter (PSI) und die zweiten repräsentativen Parameter (PIE) als Funktion einer unterschiedlichen Vergleichspriorität (Pconf) zu vergleichen.

14. Identifizierungssystem nach einem der Ansprüche 10 bis 13, wobei der Schritt zum Minimieren der Bewertung (R) umfasst:

   • (f12) Vergleichen des Abweichungswinkels ($\alpha_i$; i=1 .. n) mit Default-Schwellenwerten von Abweichungswinkeln (S1$\alpha$; S2$\alpha$) enthaltend einen ersten Default-Abweichungswinkelschwellenwert (S1$\alpha$) und einen zweiten Default-Schwellenwert von Abweichungswinkeln (S2$\alpha$), wo (S1$\alpha$) < (S2a).

**Revendications**

1. Procédé mis en œuvre par ordinateur pour identifier, chez un patient, au moins un implant dentaire (MI) pour un site d'implant (SI), comprenant les étapes de :

   (f1) simuler graphiquement, via une tomodensitométrie (TAC), une anatomie d'une prothèse dentaire (PD) envisagée, d'un site édenté (LR) respectif correspondant à une dent identifiée (Tn) et d'un os maxillaire (OM), dans lequel ladite prothèse dentaire (PD) envisagée peut être couplée à un implant dentaire (ID) pouvant être introduit dans l'os maxillaire (OM) ;

   (f2) calculer un axe prothétique (PI) idéal pour ladite prothèse dentaire (PD) envisagée sur la base de ladite tomodensitométrie (TAC), dans lequel ledit axe prothétique (PI) idéal est un axe croisant un point idéal de la prothèse dentaire (PD) envisagée en fonction du type de dent (Tn) considéré ;

   (f3) calculer une première distance cervicale (CT) et une première distance apicale (ET) dudit os maxillaire (OM) en correspondance dudit site édenté (LR) sur la base de ladite tomodensitométrie (TAC), dans lequel les distances sont calculées dans une première direction par rapport audit os maxillaire (OM), dans lequel ladite première distance cervicale (CT) est détectée en correspondance d'une première partie cervicale (q1) par rapport à une partie de référence ;

   (f4) calculer une seconde distance cervicale (CW) et une seconde distance apicale (EW) dudit os maxillaire (OM) en correspondance dudit site édenté (LR) sur la base de ladite tomodensitométrie (TAC), dans lequel les distances sont calculées dans une seconde direction par rapport à l'os maxillaire OM ;

   (f5) calculer un axe chirurgical (CI) idéal sur ledit os maxillaire (OM) en tant qu'axe passant par deux points, un premier (Cx) obtenu comme étant une intersection entre des segments représentatifs des distances cervicales (CT, CW), et un second (Ex) obtenu comme étant une intersection entre des segments représentatifs des distances apicales (ET, EW), dans lequel ledit axe chirurgical (CI) idéal est un axe configuré pour réaliser une réduction idéale de moitié de la quantité osseuse de l'os maxillaire (OM) en correspondance du site édenté (LR),

   dans lequel l'axe prothétique (PI) idéal et l'axe chirurgical (CI) idéal déterminent un système de référence (REF_ID) pour ledit implant dentaire (ID),
   dans lequel ledit axe prothétique (PI) idéal et ledit axe chirurgical (CI) idéal sont décalés selon un angle de déviation (a) ;

   (f6) calculer une hauteur osseuse (HR) dudit os maxillaire (OM) comme étant une distance sur ledit axe chirurgical (CI) idéal entre ladite première distance cervicale (CT) et ladite première distance apicale (ET), dans lequel ladite hauteur osseuse (HR) est représentative d'une disponibilité osseuse dudit os maxillaire (OM) en correspondance dudit site édenté (LR) ;

   (f7) déplacer ledit axe chirurgical (CI) idéal de façon à déterminer un axe de compromis chirurgical (CI_i ; i = 1..n) correspondant à un angle de déviation variable ($\alpha_i$ ; i = 1..n) par rapport audit axe prothétique (PI) idéal ;

(f8) simuler un site d'implant (SI) optimisé dans ledit os maxillaire (OM), dans lequel ledit site d'implant (SI) optimisé a une forme volumétrique (VSI) au moins partiellement cylindrique inscrite dans un volume délimité (V) prévu,

> ladite simulation étant réalisée au moins en fonction :
>
>> o de ladite dent identifiée (Tn) ;
>> o d'une dite première distance cervicale (CTi) et d'une dite première distance apicale (ETi) ;
>> o d'une dite seconde distance cervicale (CWi) et d'une dite seconde distance apicale (EWi) ;
>> o d'une dite première hauteur osseuse (HRi) ;
>> o dudit angle de déviation variable (αi) ;
>
> dans lequel le site d'implant (SI) optimisé est de plus simulé en fonction de ses premiers paramètres représentatifs (PSI) comprenant au moins :
>
>> o un diamètre (DTi) de ladite forme volumétrique (VSI) au moins partiellement cylindrique ;
>> o une hauteur (LIi) de ladite forme volumétrique (VSI) au moins partiellement cylindrique ;
>
> dans lequel lesdits premiers paramètres représentatifs (PSI) ont des valeurs variant en fonction dudit angle de déviation variable (αi) ;

(f9) fournir une base de données (BD_IE) comprenant des seconds paramètres représentatifs (PIE) d'implants dentaires existants (IE) ;

(f10) comparer lesdits premiers paramètres représentatifs (PSI) auxdits seconds paramètres représentatifs (PIE) ;

(f11) identifier un ou plusieurs implants dentaires (MIi) pour ledit site d'implant (SI) optimisé en fonction de la comparaison entre lesdits premiers paramètres représentatifs (PSI) et lesdits seconds paramètres représentatifs (PIE),

> dans lequel :
>
>> - dans ladite étape (f8) de simulation dudit site d'implant (SI), lesdits premiers paramètres représentatifs (PSI) comprennent de plus :
>> o une ou plusieurs valeurs d'évaluation (Rai, RDT, RLI, RRLRC, RPO, RDO) dudit site d'implant (SI) optimisé définies en fonction d'une comparaison entre lesdits premiers paramètres représentatifs (PSI) et des valeurs de seuil de référence ;

dans lequel ledit site d'implant (SI) optimisé est identifié avec une évaluation (R) définie comme une fonction combinée desdites valeurs d'évaluation (Rai, RDT, RLI, RRLRC, RPO, RDO),

dans lequel ladite étape (f11) identifie un ou plusieurs implants dentaires (BMIi) optimaux en fonction de la comparaison entre lesdits premiers paramètres représentatifs (PSI) et lesdits seconds paramètres représentatifs (PIE) lorsque ladite évaluation (R) est minimisée,

dans lequel lesdits premiers paramètres représentatifs (PSI) et lesdits seconds paramètres représentatifs (PIE) comprennent respectivement ledit diamètre (DTi) de ladite forme volumétrique (VSI) au moins partiellement cylindrique et ledit diamètre d'implant (EDTi) pour l'implant existant (IE).

2. Procédé mis en œuvre par ordinateur selon la revendication 1, dans lequel :

> lesdits premiers paramètres représentatifs (PSI) et lesdits seconds paramètres représentatifs (PIE) comprennent respectivement ledit diamètre (DTi) de ladite forme volumétrique (VSI) au moins partiellement cylindrique et ledit diamètre d'implant (EDTi) pour l'implant existant (IE) obtenus avec les sous-étapes de :
>
>> - calculer une mesure minimale (MIN_L) entre CT, MT, ET et CW, MW, EW
>> - calculer ledit diamètre (DTi ; EDTi) en fonction de la mesure minimale calculée (MIN_L) et d'une première valeur de seuil de diamètre prédéfinie (S_D1) ;
>> - vérifier que (DTi) ou (EDTi) est à une distance > = à un premier seuil d'épaisseur (S_VE) par rapport au pic vestibulaire (PV) ;
>> - vérifier que (DTi) ou (EDTi) est à une distance > = à un second seuil d'épaisseur (S_PL) par rapport au pic lingual (PL)
>
> et/ou
> dans lequel lesdits premiers paramètres représentatifs (PSI) et lesdits seconds paramètres représentatifs (PIE) comprennent respectivement ladite hauteur (LIi) de ladite forme volumétrique au moins partiellement cylindrique et ladite hauteur d'implant (ELIi) pour l'implant existant (IE), obtenues en fonction d'une valeur de hauteur osseuse (HR_i)

et/ou

dans lequel lesdits premiers paramètres représentatifs (PSI) et lesdits seconds paramètres représentatifs (PIE) comprennent un rapport couronne/racine

et/ou

dans lequel lesdits premiers paramètres représentatifs (PSI) et lesdits seconds paramètres représentatifs (PIE) comprennent un pourcentage (PO) d'implant à l'intérieur de l'os maxillaire (OM)

et/ou

dans lequel lesdits premiers paramètres représentatifs (PSI) et lesdits seconds paramètres représentatifs (PIE) comprennent une densité osseuse (DO).

3. Procédé mis en œuvre par ordinateur selon l'une quelconque des revendications précédentes, dans lequel la base de données (BD_IE) comprend lesdits seconds paramètres représentatifs (PID) représentatifs :

 o d'un identifiant de dent (ETn) pour un implant existant (IE) ;
 o d'un diamètre d'implant (EDTi) pour un implant existant (IE) ;
 o d'une hauteur d'implant (ELIi) pour un implant existant (IE) ;
 o d'un angle de déviation (Eαi) pour un implant existant (IE) ;
 o d'un identifiant d'évaluation (ERi) pour un implant existant (IE) ;
 o du fabricant de l'implant ;
 o du modèle de l'implant.

4. Procédé mis en œuvre par ordinateur selon l'une quelconque des revendications précédentes, dans lequel ladite comparaison entre lesdits premiers paramètres représentatifs (PSI) et lesdits seconds paramètres représentatifs (PIE) est effectuée en fonction d'une priorité de comparaison différente (Pconf).

5. Procédé mis en œuvre par ordinateur selon l'une quelconque des revendications 1 à 4, dans lequel ladite étape de minimisation de ladite évaluation (R) comprend :
o (f12) la comparaison dudit angle de déviation (αi ; i = 1..n) aux valeurs de seuil prédéfinies d'angles de déviation (S1α ; S2α) comprenant une première valeur de seuil d'angle de déviation prédéfinie (S1α) et une deuxième valeur de seuil d'angle de déviation prédéfinie (S2α), où (S1α) < (S2α).

6. Procédé mis en œuvre par ordinateur selon la revendication 5, dans lequel ladite étape (f12) de comparaison dudit angle de déviation (αi ; i = 1..n) comprend les étapes de :

 (f6) recalculer ladite épaisseur osseuse (HR_i) ;
 (f7) déplacer ledit axe chirurgical (CI, CI_i) par rapport audit axe prothétique (PI_i) idéal maintenu dans une position fixe, de façon à provoquer une réduction dudit angle de déviation variable (αi ; i = 1..n) ;
 dans lequel les étapes décrites sont exécutées tant que l'angle de déviation variable (αi) reste supérieur à ladite première valeur de seuil par défaut de l'angle de déviation (S1α), ledit angle de déviation (αi ; i = 1..n) étant minimisé lorsqu'il est inférieur à ladite première valeur de seuil par défaut de l'angle de déviation (S1α).

7. Procédé mis en œuvre par ordinateur selon la revendication 6, comprenant les étapes de :

 - (f13) attribuer une évaluation (Rαi) audit site d'implant (SI) en fonction de ladite étape (f12) de comparaison dudit angle de déviation variable (αi ; i = 1..n) avec la valeur de seuil par défaut de l'angle de déviation (S1α), dans lequel ladite évaluation (Rαi) diminue lorsque l'angle de déviation (αi) diminue et tend vers zéro lorsque l'angle de déviation (αi) tend vers sa valeur optimisée, dans lequel ladite étape (f13) d'attribution d'une évaluation comprend les étapes de :
 - (f6) déplacer l'axe chirurgical (CI, CI_i)
 - (f7) calculer une hauteur osseuse (HR_i ; i = 1..n) dudit os ;
 - (f8) recalculer (CTi, ETi, CWi, EWi) en fonction dudit angle de déviation variable (αi ; i = 1..n),
 - identifier un site d'implant (SI) optimal correspondant ;
 - identifier les implants dentaires optimaux (BMI) correspondants.

8. Procédé mis en œuvre par ordinateur selon l'une quelconque des revendications 3 à 7, comprenant l'étape consistant à attribuer une évaluation à ladite mesure d'un diamètre (DTi) ou (EDTi) dans lequel :

 - Si ledit diamètre (DTi) ou (EDTi) est < à la seconde valeur de seuil de diamètre par défaut (S_D2) → évaluation = - 1
 - Si ledit diamètre (DTi) ou (EDTi) est < à la troisième valeur de seuil de diamètre par défaut (S_D3) → évaluation = - 2
 et/ou

  attribuer une évaluation à ladite mesure de hauteur (LIi ; ELIi) en fonction d'une première valeur de seuil de hauteur par défaut (SL_1)
  et/ou
  attribuer une évaluation (R_RI_RC) audit rapport couronne/racine (RL_RC), dans lequel

si RL / CL > = à un premier seuil (SRLRC1), (en particulier = 1) → évaluation = 0
si (RL / CL) < (SRLRC1) → évaluation - 1 ;
si RL / CL < (SRLRC2) < (SRLRC1), (en particulier SRLRC2 = 0,75) → évaluation - 2 ;
et/ou

attribuer une évaluation (R_PO) au pourcentage de l'implant à l'intérieur de l'os (PO), dans lequel :

si (PO) = 100 % → évaluation = 0 ;
si le premier seuil (PO1) (95 %) < PO < second seuil (PO2) (99 %) → évaluation - 1 ;
si PO < premier seuil (PO1) (95 %) → évaluation - 2 et/ou

attribuer une évaluation (R_DO) à ladite densité osseuse (PO), dans lequel :

si DO > seuil de densité (SDO) → évaluation = 0
si DO < seuil de densité (SDO) → évaluation = - 1.

9. Programme d'ordinateur configuré pour, lorsqu'il est utilisé, exécuter le procédé mis en œuvre par ordinateur selon l'une des revendications précédentes.

10. Système d'identification d'au moins un implant dentaire (MI) pour un site d'implant (SI) incluant :

des moyens graphiques de simulation (1) mis en œuvre par ordinateur configurés pour simuler graphiquement via une tomodensitométrie (TAC) une anatomie d'une prothèse dentaire (PD) fournie, d'un site édenté (LR) respectif correspondant à une dent identifiée (Tn) et d'un os maxillaire (OM), dans lequel ladite prothèse dentaire (PD) fournie peut être couplée à un implant dentaire (ID) pouvant être introduit dans l'os maxillaire (OM) ;
une unité de traitement (10) configurée pour simuler un site d'implant (SI) optimisé à partir de la simulation de l'anatomie de la prothèse dentaire (PD) fournie, dans lequel l'unité de traitement 10 comprend :

- un premier module de calcul (101) configuré pour calculer un axe prothétique (PI) idéal pour la prothèse dentaire (PD) fournie sur la base de ladite tomodensitométrie (TAC), dans lequel ledit axe prothétique (PI) idéal est un axe croisant un point idéal de la prothèse dentaire (PD) fournie en fonction du type de dent (Tn) considéré ;
- un deuxième module de calcul (102) configuré pour calculer une première distance cervicale (CT) et une première distance apicale (ET) dudit os maxillaire (OM) en correspondance dudit site édenté (LR) sur la base de ladite tomodensitométrie (TAC), dans lequel les distances sont calculées dans une première direction par rapport audit os maxillaire (OM), dans lequel ladite première distance cervicale (CT) est détectée en correspondance d'une première partie cervicale (q1) par rapport à une partie de référence ;
- un troisième module de calcul (103) configuré pour calculer une seconde distance cervicale (CW) et une seconde distance apicale (EW) dudit os maxillaire (OM) en correspondance dudit site édenté (LR) sur la base de ladite tomodensitométrie (TAC), dans lequel les distances sont calculées dans une seconde direction par rapport à l'os maxillaire (OM) ;
- un quatrième module de calcul (104) configuré pour calculer un axe chirurgical (CI) idéal sur ledit os maxillaire (OM) comme étant un axe passant par deux points, un premier (Cx) obtenu en comme étant une intersection entre des segments représentatifs des distances cervicales (CT, CW), et un second (Ex) obtenu comme étant une intersection entre des segments représentatifs des distances apicales (ET, EW), dans lequel ledit axe chirurgical (CI) idéal est un axe configuré pour réaliser une réduction idéale de moitié de la quantité osseuse de l'os maxillaire (OM) en correspondance du site édenté (LR) ;

dans lequel l'axe prothétique (PI) idéal et l'axe chirurgical (CI) idéal déterminent un système de référence (REF_ID) pour ledit implant dentaire (ID) ;
dans lequel ledit axe prothétique (PI) idéal et ledit axe chirurgical (CI) idéal sont décalés selon un angle de déviation (α) ;

- un cinquième module de calcul (105) configuré pour calculer une hauteur osseuse (HR) dudit os maxillaire (OM) comme étant une distance sur ledit axe chirurgical (CI) idéal entre ladite première distance cervicale (CT) et ladite première distance apicale (ET), dans lequel ladite hauteur osseuse (HR) est représentative d'une disponibilité osseuse dudit os maxillaire (OM) en correspondance dudit site édenté (LR) ;
- un module de déplacement (106) configuré pour déplacer ledit axe chirurgical (CI) idéal en déterminant un axe chirurgical de compromis (CI_i ; i = 1..n) correspondant à un angle de déviation variable ($\alpha$i ; i = 1..n) par rapport audit axe prothétique (PI) idéal ;
- un module de simulation (107B) configuré pour simuler ledit site d'implant (SI) optimisé dans ledit os maxillaire (OM), dans lequel ledit site d'implant (SI) optimisé présente une forme volumétrique (VSI) au moins partiellement cylindrique inscrite à l'intérieur d'un volume délimité (V) prévu, ladite simulation étant réalisée au moins en fonction :

    • de ladite dent identifiée (Tn) ;
    • d'une dite première distance cervicale (CTi) et d'une dite première distance apicale (ETi) ;
    • d'une dite seconde distance cervicale (CWi) et d'une dite seconde distance apicale (EWi) ;
    • d'une dite première hauteur osseuse (HRi) ;
    • dudit angle de déviation variable ($\alpha$i) ;
- dans lequel le site d'implant (SI) optimisé est au moins simulé en fonction de ses premiers paramètres représentatifs (PSI) incluant au moins :

    • un diamètre (DTi) de ladite forme volumétrique (VSI) au moins partiellement cylindrique
    • une hauteur (LIi) de ladite forme volumétrique (VSI) au moins partiellement cylindrique ;

dans lequel lesdits premiers paramètres représentatifs (PSI) ont des valeurs variables en fonction dudit angle de déviation variable ($\alpha$i) ;

    - une base de données (BD_IE) incluant des seconds paramètres représentatifs (PIE) d'implants dentaires (IE) existants, une seconde unité de traitement (20) comprenant :
    - un module de comparaison (201) configuré pour comparer lesdits premiers paramètres représentatifs (PSI) auxdits seconds paramètres représentatifs (PIE) ;
    - un premier module d'identification (202) configuré pour identifier un ou plusieurs implants dentaires (MIi) pour ledit site d'implant (SI) optimisé en fonction de la comparaison effectuée par le module de comparaison (201) ;

dans lequel lesdits premiers paramètres représentatifs (PSI) comprennent de plus une ou plusieurs valeurs d'évaluation (Rai, RDT, RLI, RRLRC, RPO, RDO) dudit site d'implant (SI) optimisé définies en fonction d'une comparaison entre les premiers paramètres représentatifs PSI et des valeurs de seuil de référence ;
dans lequel le site d'implant SI optimisé est identifié avec une évaluation R définie comme une fonction combinée desdites valeurs d'évaluation (Rai, RDT, RLI, RRLRC, RPO, RDO),

    - un second module d'identification (203) configuré pour identifier un ou plusieurs implants dentaires optimaux (BMIi) en fonction de la comparaison effectuée par ledit module de comparaison (201) lorsque ladite évaluation (R) est minimisée,

dans lequel lesdits premiers paramètres représentatifs (PSI) et lesdits seconds paramètres représentatifs (PIE) comprennent respectivement ledit diamètre (DTi) de ladite forme volumétrique (VSI) au moins partiellement cylindrique et ledit diamètre d'implant (EDTi) pour chaque implant existant (IE).

11. Système d'identification selon la revendication 10, dans lequel lesdits premiers paramètres représentatifs (PSI) et lesdits seconds paramètres représentatifs (PIE) comprennent respectivement ledit diamètre (DTi) de ladite forme volumétrique (VSI) au moins partiellement cylindrique et ledit diamètre d'implant (EDTi) pour chaque implant existant (IE) obtenus avec les sous-étapes de :

    - calculer une mesure minimale (MIN_L) entre CT, MT, ET et CW, MW, EW
    - calculer ledit diamètre (DTi ; EDTi) en fonction de la mesure minimale calculée (MIN_L) et d'une première valeur de seuil de diamètre par défaut (S_D1) ;
    - vérifier que (DTi) ou (EDTi) est à une distance > = d'un premier seuil d'épaisseur (S_VE) par rapport au pic vestibulaire (PV) ;
    - vérifier que (DTi) ou (EDTi) est à une distance> = d'un second seuil d'épaisseur (S_PL) par rapport au pic lingual (PL)
    et/ou

    dans lequel lesdits premiers paramètres représentatifs (PSI) et lesdits seconds paramètres représentatifs (PIE) comprennent respectivement la hauteur (LIi) de ladite forme volumétrique au moins partiellement cylindrique et ladite hauteur d'implant (ELIi) pour chaque implant existant (IE), obtenues en fonction d'une valeur de hauteur osseuse (HR_i)
    et/ou
    dans lequel lesdits premiers paramètres représentatifs (PSI) et lesdits seconds paramètres représentatifs (PIE) comprennent un rapport couronne/racine
et/ou
dans lequel lesdits premiers paramètres représentatifs (PSI) et lesdits seconds paramètres représentatifs (PIE) comprennent un pourcentage (PO) de l'implant à l'intérieur de l'os maxillaire (OM)
et/ou
dans lequel lesdits premiers paramètres représentatifs (PSI) et lesdits seconds paramètres représentatifs (PIE) comprennent une densité osseuse (DO).

12. Système d'identification selon l'une quelconque des revendications 10 à 11, dans lequel la base de données (BD_IE) comprend lesdits seconds paramètres représentatifs (PID) représentatifs :

    • d'un identifiant de dent (ETn) pour chaque implant existant (IE) ;
    • d'un diamètre d'implant (EDTi) pour chaque implant existant (IE) ;
    • d'une hauteur d'implant (ELIi) pour chaque implant existant (IE) ;
    • d'un angle de déviation (E$\alpha$i) pour chaque implant existant (IE) ;
    • d'un identifiant d'évaluation (ERi) pour chaque implant existant (IE) ;
    • du fabricant de l'implant ;
    • du modèle d'implant.

13. Système d'identification selon l'une quelconque des revendications 10 à 12, dans lequel ledit module de comparaison (201) est configuré pour comparer lesdits premiers paramètres représentatifs (PSI) et lesdits seconds paramètres représentatifs (PIE) en fonction d'une priorité de comparaison différente (Pconf).

14. Système d'identification selon l'une quelconque des revendications 10 à 13, dans lequel ladite étape de minimisation de ladite évaluation (R) comprend :

    • (f12) comparer ledit angle de déviation ($\alpha$i ; i = 1..n) aux valeurs de seuil d'angles de déviation par défaut (S1$\alpha$ ; S2$\alpha$) incluant une première valeur de seuil d'angle de déviation par défaut (S1$\alpha$) et une seconde valeur de seuil d'angles de déviation par défaut (S2$\alpha$), où (S1$\alpha$) < (S2$\alpha$).

GINGIVA

LR

OM

Z

Y

X

SI(VSI)

ID

Fig.1

EP 3 582 715 B1

Fig.4

Fig.8

EP 3 582 715 B1

Fig.2 Fig.3

EP 3 582 715 B1

# Fig.5

CI

PI

ALVEOLAR
CREST

CROWN CO

Pt1

DENTAL
PROSTHESIS PD

CORTICAL

SO

CERVICAL MARGIN
OF THE ALVEOLAR
CREST

CORTICAL

α

APICAL MARGIN
OF THE ALVEOLAR
CREST

GINGIVA
THICKNESS

MAXILLARY
BONE OM

LOWER ALVEOLAR
NERVE NAI

CROWN CO

CI

PROSTHESIS PD

UPPER BONE MARGIN

CERVICAL
CT MEASURE

GINGIVA

Q1

HR=Q1-Q2 su CI_3

MT

Q3

APICAL
MEASURE OF THE
MANDIBULAR BONE

ET

Q2

HR

A-A'

LOWER ALVEOLAR
NERVE NAI

MAXILLARY
BONE
OM

Fig.6

Fig.7

Fig.7A

EP 3 582 715 B1

| | |
|---|---|
| Q1 | CT,CX,CW |
| Q3 | MX,MT,MW |
| Q2 | EX,EW,ET |

Fig.9

CI_3=A0

CI_2

CI_1

DISPLACEMENT
REFERENCE

α3

α2

REF_ID

S2α

S1α

α1

PI

α1>S2α ⟶ CI_1

S1α<α2<S2α ⟶ CI_2

α3<S1α ⟶ CI_3=A0

BMI

Fig.10A

Fig.10B
CASE I  GH>S_GH

Fig.10C
CASE II  GH<S_GH

Fig.11

Fig.13

Fig.14

EP 3 582 715 B1

Fig.12

Fig.15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2012239364 A1